# EUROPEAN PATENT APPLICATION

(11) **EP 3 382 005 A2**
(43) Date of publication of application: **03.10.2018**
(21) Application number: 18165193.6
(22) Date of filing: 29.03.2018
(51) Int. Cl.: C12M 3/00, G01N 33/50, C12M 1/04

(54) **EX VIVO MODEL FOR NEURODEGENERATIVE DISEASES**

(30) Priority: 29.03.2017 GB 201704983
(71) Applicant: Neuro-Bio Ltd, Abingdon, Oxfordshire OX14 3DB (GB)
(72) Inventor: GREENFIELD, Susan, Abingdon, Oxforshire OX14 3DB (GB); BRAI, Emanuele, Abingdon, Oxfordshire OX14 3DB (GB)
(74) Representative: Hutter, Anton

(57) **Abstract**

The invention relates to animal models, and in particular to novel *ex-vivo* animal models for investigating the underlying mechanisms occurring in neurodegenerative disorders, such as Alzheimer's disease, Parkinson's disease or Motor Neurone Disease. The invention extends to apparatus, methods and assays for providing such models, as well as for testing pharmacological compounds, which may regulate neurological processes, and for drug screening for use in treating neurodegenerative disease. The invention also relates to novel polypeptides, peptidomimetics and compositions, for use in the assays and apparatus used to investigate neurodegenerative disorders, and for drug screening. The invention also relates to apparatus and methods for animal models for investigating neuroregeneration.

SEQUENCE LISTING

<110>  Neuro-Bio Ltd

<120>  Animal Model

<130>  79153EP1

<160>  5

<170>  PatentIn version 3.5

<210>  1
<211>  614
<212>  PRT
<213>  Homo sapiens

<400>  1

Met Arg Pro Pro Gln Cys Leu Leu His Thr Pro Ser Leu Ala Ser Pro
1               5                   10                  15

Leu Leu Leu Leu Leu Leu Trp Leu Leu Gly Gly Gly Val Gly Ala Glu
            20                  25                  30

Gly Arg Glu Asp Ala Glu Leu Leu Val Thr Val Arg Gly Gly Arg Leu
        35                  40                  45

Arg Gly Ile Arg Leu Lys Thr Pro Gly Gly Pro Val Ser Ala Phe Leu
    50                  55                  60

Gly Ile Pro Phe Ala Glu Pro Pro Met Gly Pro Arg Arg Phe Leu Pro
65                  70                  75                  80

Pro Glu Pro Lys Gln Pro Trp Ser Gly Val Val Asp Ala Thr Thr Phe
                85                  90                  95

Gln Ser Val Cys Tyr Gln Tyr Val Asp Thr Leu Tyr Pro Gly Phe Glu
            100                 105                 110

Gly Thr Glu Met Trp Asn Pro Asn Arg Glu Leu Ser Glu Asp Cys Leu
        115                 120                 125

Tyr Leu Asn Val Trp Thr Pro Tyr Pro Arg Pro Thr Ser Pro Thr Pro
    130                 135                 140

Val Leu Val Trp Ile Tyr Gly Gly Gly Phe Tyr Ser Gly Ala Ser Ser
145                 150                 155                 160

Leu Asp Val Tyr Asp Gly Arg Phe Leu Val Gln Ala Glu Arg Thr Val
                165                 170                 175

Leu Val Ser Met Asn Tyr Arg Val Gly Ala Phe Gly Phe Leu Ala Leu
            180                 185                 190

Pro Gly Ser Arg Glu Ala Pro Gly Asn Val Gly Leu Leu Asp Gln Arg
        195                 200                 205

Leu Ala Leu Gln Trp Val Gln Glu Asn Val Ala Ala Phe Gly Gly Asp
    210                 215                 220

Pro Thr Ser Val Thr Leu Phe Gly Glu Ser Ala Gly Ala Ala Ser Val
225                 230                 235                 240

Gly Met His Leu Leu Ser Pro Pro Ser Arg Gly Leu Phe His Arg Ala
                245                 250                 255

Val Leu Gln Ser Gly Ala Pro Asn Gly Pro Trp Ala Thr Val Gly Met
            260                 265                 270

Gly Glu Ala Arg Arg Arg Ala Thr Gln Leu Ala His Leu Val Gly Cys
        275                 280                 285

Pro Pro Gly Gly Thr Gly Gly Asn Asp Thr Glu Leu Val Ala Cys Leu
    290                 295                 300

Arg Thr Arg Pro Ala Gln Val Leu Val Asn His Glu Trp His Val Leu
305                 310                 315                 320

Pro Gln Glu Ser Val Phe Arg Phe Ser Phe Val Pro Val Val Asp Gly
                325                 330                 335

Asp Phe Leu Ser Asp Thr Pro Glu Ala Leu Ile Asn Ala Gly Asp Phe
            340                 345                 350

His Gly Leu Gln Val Leu Val Gly Val Val Lys Asp Glu Gly Ser Tyr
        355                 360                 365

Phe Leu Val Tyr Gly Ala Pro Gly Phe Ser Lys Asp Asn Glu Ser Leu
    370                 375                 380

Ile Ser Arg Ala Glu Phe Leu Ala Gly Val Arg Val Gly Val Pro Gln
385                 390                 395                 400

Val Ser Asp Leu Ala Ala Glu Ala Val Val Leu His Tyr Thr Asp Trp
                405                 410                 415

Leu His Pro Glu Asp Pro Ala Arg Leu Arg Glu Ala Leu Ser Asp Val
            420                 425                 430

Val Gly Asp His Asn Val Val Cys Pro Val Ala Gln Leu Ala Gly Arg
        435                 440                 445

Leu Ala Ala Gln Gly Ala Arg Val Tyr Ala Tyr Val Phe Glu His Arg
    450                 455                 460

Ala Ser Thr Leu Ser Trp Pro Leu Trp Met Gly Val Pro His Gly Tyr
465                 470                 475                 480

Glu Ile Glu Phe Ile Phe Gly Ile Pro Leu Asp Pro Ser Arg Asn Tyr
                485                 490                 495

Thr Ala Glu Glu Lys Ile Phe Ala Gln Arg Leu Met Arg Tyr Trp Ala
            500                 505                 510

Asn Phe Ala Arg Thr Gly Asp Pro Asn Glu Pro Arg Asp Pro Lys Ala
        515                 520                 525

Pro Gln Trp Pro Pro Tyr Thr Ala Gly Ala Gln Gln Tyr Val Ser Leu
    530                 535                 540

Asp Leu Arg Pro Leu Glu Val Arg Arg Gly Leu Arg Ala Gln Ala Cys
545                 550                 555                 560

Ala Phe Trp Asn Arg Phe Leu Pro Lys Leu Leu Ser Ala Thr Asp Thr
                565                 570                 575

Leu Asp Glu Ala Glu Arg Gln Trp Lys Ala Glu Phe His Arg Trp Ser
            580                 585                 590

Ser Tyr Met Val His Trp Lys Asn Gln Phe Asp His Tyr Ser Lys Gln
        595                 600                 605

Asp Arg Cys Ser Asp Leu
    610

<210>  2
<211>  42
<212>  PRT
<213>  Homo sapiens

<400>  2

<210>  3
<211>  30
<212>  PRT
<213>  Homo sapiens

<400>  3

Lys Ala Glu Phe His Arg Trp Ser Ser Tyr Met Val His Trp Lys Asn
1               5                   10                  15

Gln Phe Asp His Tyr Ser Lys Gln Asp Arg Cys Ser Asp Leu
            20                  25                  30

<210>  4
<211>  14
<212>  PRT
<213>  Homo sapiens

<400>  4

Ala Glu Phe His Arg Trp Ser Ser Tyr Met Val His Trp Lys
1               5                   10

<210>  5
<211>  15
<212>  PRT
<213>  Homo sapiens

<400>  5

Asn Gln Phe Asp His Tyr Ser Lys Gln Asp Arg Cys Ser Asp Leu
1               5                   10                  15

## Description

The invention relates to animal models, and in particular to novel *ex-vivo* animal models for investigating the underlying mechanisms occurring in neurodegenerative disorders, such as Alzheimer's disease, Parkinson's disease or Motor Neurone Disease. The invention extends to apparatus, methods and assays for providing such models, as well as for testing pharmacological compounds, which may regulate neurological processes, and for drug screening for use in treating neurodegenerative disease. The invention also relates to novel polypeptides, peptidomimetics and compositions, for use in the assays and apparatus used to investigate neurodegenerative disorders, and for drug screening. The invention also relates to apparatus and methods for animal models for investigating neuroregeneration.

Alzheimer's disease (AD) is the most common form of dementia, but the primary events promoting this disorder remain still unravelled. The most popular "amyloid hypothesis" is now being increasingly challenged [1, 2] and an alternative theory compatible with all clinical features is needed. One such different approach focuses on the distinguishing properties of the neurons that are selectively and primarily vulnerable in AD [3-8]. They constitute a continuous hub of adjacent cell groups, extending from the basal forebrain (BF) to midbrain and brainstem, which send projections to several brain areas, such as cortex, hippocampus and olfactory bulb [9]. Despite a heterogeneity of transmitters within this core of susceptible cells, an interesting common feature is that they contain the enzyme acetylcholinesterase, now established to exert a non-cholinergic function [10, 11]. This non-enzymatic role modulates calcium influx into neurons [10, 11] and, hence, it can be trophic or toxic, depending on dose, availability and neuronal age.

In AD, therefore, cell death could be caused by this non-hydrolytic action [10-12] and more specifically, via a 14mer peptide cleaved from the AChE C-terminus and included within a more stable, larger peptide, T30 [12]. In addition, the AChE-derived peptide sequence shares a strong homology with Aβ [10,11], is doubled in AD brains and drives production of p-Tau and Aβ in PC12 cell lines [11] via its binding to the α7-nAChR and consequent modulation of the calcium entry [11-14]. Furthermore, this nicotinic receptor is upregulated upon T30 exposure in GH4-hα7 cells [13].

Currently there is no widely accepted animal model which reproduces the full pathological profile of a neurodegenerative disorder, such as Alzheimer's disease (AD), since the basic mechanisms of neurodegeneration are still poorly understood. There is therefore a need for an improved model/assay and corresponding kit, which enables the accurate study of neurodegenerative disorders.

As discussed herein, the inventors have developed a new hypothesis which they believe accounts for the aberrant processes characterizing Alzheimer's disease, based on the interaction between the α7 nicotinic acetylcholine receptor (α7-nAChR) and the recently discovered toxic peptide, which is cleaved from the acetylcholinesterase (AChE) C-terminus, i.e. T30. Based on this hypothesis, they have created a novel approach using AN *ex-vivo* model (*ex-vivo* brain slices), which can be used to study neurodegenerative disorders, in a more physiological scenario than cell cultures, by evaluating the effects of toxic compounds (e.g. T30), on modulating the expression of its endogenous bioactive peptide, T14, and some proteins affected in neurodegenerative processes, such as α7-nAChR, phosphorylated Tau (p-Tau) and amyloid beta (Aβ). The inventors have also designed and tested a novel peptidomimetic, Tri01, which, in cell cultures, exhibits toxic effects, similar to those displayed by T30. The model can also be used to investigate neuroregeneration using trophic factors.

Adopting a new approach on *ex-vivo* model, which combines the advantages of both *in-vivo* and *in-vitro* preparations, the inventors incubated rat brain coronal sections, containing the basal forebrain (BF), in aCSF (artificial cerebrospinal fluid) alone or with a toxic compound, such as the AChE-derived peptide, "T30", or the peptidomimetic, "Tri01". The inventors investigated the T30/Tri01-mediated modifications on three Alzheimer's disease hallmarks, namely α7-nAchR, p-Tau and Aβ.

Western blot results show that the levels of T14, α7-nAChR, p-Tau and Aβ are differentially expressed upon the T30-peptide administration, in a selective site-dependent manner. In conclusion, this methodology demonstrates the action of a novel peptide (T30) in triggering an Alzheimer's disease-like profile and more generally suggests a new approach, which maintains several aspects of the *in-vivo* context, for monitoring and manipulating neurochemical phenomena contributing to neurodegeneration, in a time-dependent and site-specific manner.

This new approach on handling *ex-vivo* brain slices allows the exploration of the early stages occurring during neurodegeneration in a more physiological context, maintaining the local neuronal circuitry of the studied region and giving the possibility to monitor its acute response. The application of this methodology could be used to examine many molecular processes, test pharmacological compounds which may regulate these processes and provide a reliable tool for drug screening, reducing whole animal experiments.

The inventors have designed, built and tested an apparatus for conducting an animal model for a neurodegenerative disease.

Thus, in a first aspect of the invention, there is provided an apparatus for carrying out an animal model for a neurodegenerative disease, the apparatus comprising:-
- a first container configured to receive a first brain section from an animal brain, and comprising incubation media for retaining viability of the brain section;
- a second container configured to receive a second brain section, which is contralateral with the first brain section, and comprising incubation media for retaining viability of the brain section; and
- oxygen feed means configured to feed oxygen into the media in the first and second containers to thereby incubate the first and second brain sections to retain their viability,
wherein the incubation media in at least one of the containers comprises a compound which contributes to, or causes, neurodegeneration of the brain section contained therein.

The inventors have also devised a method for producing an animal model for neurodegenerative disease.

Hence, in a second aspect, there is provided a method of providing an animal model for a neurodegenerative disease, the method comprising:-
- removing a region of an animal brain, and dividing the brain region into corresponding first and second contralateral brain sections;
- incubating the first and second brain sections to retain their viability; and
- contacting at least one of the brain sections with a compound which contributes to, or causes, neurodegeneration of the brain section.

The animal model may be *in-vitro.* However, most preferably the animal model is an *ex-vivo* model.

An important advantage of the apparatus and methods of the invention is that they do not involve any pre-treatment of the brain tissue. Following decapitation of the animal, within a short period of time (e.g. about four minutes), the first and second brain sections (i.e. contralateral counterparts of each other), which are still living and viable, are incubated either in the presence of a control oxygenated solution, or in the presence of the toxic compound (such as T14, T15, T30 or a Tri01 peptidomimetic), which causes or at least contributes to neurodegeneration of the brain section. Furthermore, the animal model allows for varying parameters (e.g. duration of the exposure to, and the concentration of, the toxic compound) and controlling them precisely in a way that would be impossible using an *in-vivo* or *in-vitro* model with a duplicate control. Unlike an *in-vivo* model, the *ex-vivo* model harnessed in the apparatus and methods of the invention would, for the first time ever, also allow a detailed study of the time course of production of β-amyloid and Tau, and the conditions promoting the living brain. Another important advantage of the invention is a significant reduction in the use of living animal for drug testing.

The apparatus and method of the invention can be used to examine many molecular processes, test pharmacological compounds which may regulate these processes and provide a reliable tool for drug screening.

Thus, in a third aspect, there is provided use of the apparatus of the first aspect to: (i) examine neurodegenerative processes; (ii) test pharmacological compounds which may modulate neurodegenerative processes; or (iii) screen neurodegenerative drugs.

The inventors have modified the apparatus and method of the invention such that the two contralateral brain sections are exposed to the toxic compound which causes neurodegeneration, and then, in one of the containers, exposing one of the treated brain sections to a test agent, and monitoring the effects to determine if the test agent prevents, inhibits or increases neurodegeneration.

Hence, in a fourth aspect, there is provided a method of identifying an agent that modulates neurodegeneration, the method comprising:
- removing a region of an animal brain, and dividing the brain region into corresponding first and second contralateral brain sections;
- contacting each brain section with a compound which contributes to, or causes, neurodegeneration of the brain sections;
- contacting one of the brain sections with a test agent prior to, simultaneously or after the compound which causes, or contributes to, neurodegeneration of the brain section; and
- determining whether the test agent modulates neurodegeneration in the brain section by comparing its effects against the brain section untreated with the test agent.

Modulation of neurodegeneration may include inhibition, prevention or increasing neurodegeneration.

In a fifth aspect, there is provided a method of identifying a candidate agent, for use in the treatment, prevention or amelioration of neurodegenerative disorder, the method comprising:
- removing a region of an animal brain, and dividing the brain region into corresponding first and second contralateral brain sections;
- contacting each brain section with a compound which contributes to, or causes, neurodegeneration of the brain sections;
- contacting one of the brain sections with a test agent prior to, simultaneously or after the compound which causes, or contributes to, neurodegeneration of the brain section; and
- determining whether the test agent inhibits or prevents neurodegeneration in the brain section by comparing its effects against the brain section untreated with the test agent, wherein inhibition or prevention of neurodegeneration compared to the control indicates that the test agent is a candidate for the treatment, prevention or amelioration of neurodegenerative disorder.

Preferably, the methods of the invention comprise detecting α7-nAchR, p-Tau and/or Aβ expressed by the first and/or second brain section. However, it will be appreciated that there are other markers of neurodegeneration that could be detected, such as GFAP (to label astroglia), CD68 (a marker for active microglial cells, involved in neuroinflammatory responses), interleukins, protein kinase family (e.g. GSK3β and CDK5), Reelin, AChE and/ChAT. Detection may be conducted using Western blot analysis or ELISA.

The apparatus or methods may be used to investigate any neurodegenerative disease selected from a group consisting of: including Alzheimer's disease; Parkinson's disease; Huntington's disease; Motor Neurone disease; Spinocerebellar type 1, type 2, and type 3; Amyotrophic Lateral Sclerosis (ALS); schizophrenia; Lewy-body dementia; and Frontotemporal Dementia. It is preferred that the model is used to study any neurological disorder associated with non-enzymatic function of AChE, in particular, for example, Alzheimer's Disease, Parkinson's Disease and Motor Neuron Disease.

However, it is especially preferred that the model is used to study Alzheimer's Disease.

As described in the Examples, a brain is preferably dissected from the animal's skull. Preferably, the brain is a non-human mammalian brain. Preferably, the brain is a rodent brain, either rat or mouse. Most preferably, rat brain is used. Preferably, the brain is cooled, for example with ice-cold media. Preferably, the brain is attached to a support surface, such as a Vibratome disc. Preferably, then contacted with cooled incubation media. Preferably, the media comprises cerebrospinal fluid (CSF), most preferably artificial cerebrospinal fluid (aCSF).

Preferably, the first and second brain sections comprise the basal forebrain. Thus, preferably the brain is sectioned into coronal slices containing the basal forebrain (BF). Preferably, the coronal slices are divided into two complementary (i.e. contralateral) hemisections. Thus, the first and second brain sections preferably each comprise contralateral hemisections comprising the basal forebrain. Preferably, the first and second brain sections are selected from a group consisting of: the rostral region of the basal forebrain; the intermediate region of the basal forebrain; and the caudal region of the basal forebrain. Hence, in the methods, the brain is sliced into three consecutive sections, which contain the rostral (slice 1), the intermediate (slice 2) and caudal (slice 3) portions of the basal forebrain. Then, each coronal section is divided in two along the midline, providing the control side and the treated one along the same anatomical plane.

In one preferred embodiment, the first and second brain sections comprise contralateral hemisections comprising the rostral region of the basal forebrain. In another preferred embodiment, the first and second brain sections comprise contralateral hemisections comprising the intermediate region of the basal forebrain. In another preferred embodiment, the first and second brain sections comprise contralateral hemisections comprising the caudal region of the basal forebrain.

In a preferred embodiment, the apparatus comprises four containers, wherein two pairs of containers are configured to each receive contralateral sections of two different regions of the basal forebrain. For example, one pair of containers may be configured to receive the rostral region, while the other pair of containers may be configured to receive the intermediate region. Alternatively, one pair of containers may be configured to receive the rostral region, while the other pair of containers may be configured to receive the caudal region. In another embodiment, one pair of containers may be configured to receive the intermediate region, while the other pair of containers may be configured to receive the caudal region.

However, in a most preferred embodiment, the apparatus comprises six containers, wherein three pairs of containers are configured to each receive contralateral sections of three different regions of the basal forebrain, i.e. one pair of containers are configured to receive the rostral region, one pair of containers are configured to receive the intermediate region, and the final pair of containers are configured to receive the caudal region.

Hence, the apparatus preferably comprises:-
- a first container configured to receive a first brain section comprising the rostral region of the basal forebrain, and a second container configured to receive a second contralateral brain section also comprising the rostral region of the basal forebrain; and/or
- a third container configured to receive a third brain section comprising the intermediate region of the basal forebrain, and a fourth container configured to receive a fourth contralateral brain section also comprising the intermediate region of the basal forebrain; and/or
- a fifth container configured to receive a fifth brain section comprising the caudal region of the basal forebrain, and a sixth container configured to receive a sixth contralateral brain section also comprising the caudal region of the basal forebrain.

It will be appreciated that the methods of the invention comprise removing regions of the animal's brain which correspond to the various regions of the basal forebrain described above. For examples, the methods may comprise removing a brain region including the forebrain, and then dividing it into separate contralateral brain hemisections which comprise the rostral region, the intermediate region and/or the caudal region of the basal forebrain, which may then be inserted into corresponding containers. Preferably, each brain section is disposed on a substrate in its container to prevent direct contact between the brain section and an inner surface of the container. The substrate may, for example, comprise filter paper.

The oxygen feed means preferably comprises a source of oxygen gas, and one or more conduits and associated valves, which are configured to feed oxygen into the incubation media that is present in each container. Preferably, one end of the conduits comprises a needle which extends into the incubation media in the container. Advantageously, this ensures that the media is fully oxygenated such that the brain sections are adequately incubated and therefore kept viable throughout the experiments. Each container preferably comprises a vent through which excess oxygen may be vented to atmosphere.

Preferably, one member of the or each pair of containers is configured to receive a contralateral brain section is a "control container" in which its brain section is contacted with the media to retain its viability, and the other member of the pair of containers is a "test container" in which its brain section is additionally treated with the compound, which contributes to, or causes, neurodegeneration.

Preferably, the brain section in the test container is exposed to an effective amount of the compound such that it contributes to, or causes, neurodegeneration. For example, the concentration of the compound may be between 0.1 µM and 1000µM, or between 0.5 µM and 500µM, or between 1µM and 100µM, or between 0.15 µM and 10µM. In a most preferred embodiment, about 2 µM of T30, Tri01 and NBP14 is used.

Preferably, the brain section in the test container is exposed to the compound, which contributes to, or causes, neurodegeneration for at least 1 hour, 2 hours, 3 hours, 4 hours, or 5 hours. Preferably, the brain section in the test container is exposed to the compound for less than 7 hours or 6 hours. Most preferably, the brain section in the test container is exposed to the compound for between 1 and 7 hours, even more preferably between 2 and 6 hours, and most preferably between 3 and 5.5 hours. In a most preferred embodiment, a maximum exposure time of about 5 hours is preferred.

The inventors have found that the control container with the control brain slice starts to show some neurodegeneration even in the absence of the toxic compound after 7 hours, and so it is preferred to limit the time of the experiment accordingly.

Preferably, the compound, which contributes to, or causes, neurodegeneration of the brain section, is an allosteric modulator of the α7 nicotinic receptor. In one preferred embodiment, the compound comprises a polypeptide, derivative or analogue thereof, comprising or consisting of an amino acid sequence derived from the C-terminus of acetylcholinesterase (AChE), or a truncation thereof. Acetylcholinesterase is a serine protease that hydrolyses acetylcholine, and will be well-known to the skilled person. The major form of acetylcholinesterase which is found in the brain is known as tailed acetylcholinesterase (T-AChE), and the protein sequence of one embodiment of human tailed acetylcholinesterase (Gen Bank: AAA68151.1) is 614 amino acids in length, and is provided herein as SEQ ID No:1, as follows:

1 mrppqcllht pslaspllll llwllgggvg aegredaell vtvrggrlrg irlktpggpv
61 saflgipfae ppmgprrflp pepkqpwsgv vdattfqsvc yqyvdtlypg fegtemwnpn
121 relsedclyl nvwtpyprpt sptpvlvwiy gggfysgass ldvydgrflv qaertvlvsm
181 nyrvgafgfl alpgsreapg nvglldqrla lqwvqenvaa fggdptsvtl fgesagaasv
241 gmhllsppsr glfhravlqs gapngpwatv gmgearrrat qlahlvgcpp ggtggndtel
301 vaclrtrpaq vlvnhewhvl pqesvfrfsf vpvvdgdfls dtpealinag dfhglqvlvg
361 vvkdegsyfl vygapgfskd neslisraef lagvrvgvpq vsdlaaeavv lhytdwlhpe
421 dparlreals dvvgdhnvvc pvaqlagrla aqgarvyayv fehrastlsw plwmgvphgy
481 eiefifgipl dpsrnytaee kifaqrlmry wanfartgdp neprdpkapq wppytagaqq
541 yvsldlrple vrrglraqac afwnrflpkl lsatdtldea erqwkaefhr wssymvhwkn
601 qfdhyskqdr csdl

[SEQ ID No:1]

The first 31 amino acid residues of SEQ ID No:1 are removed while the protein is released, thereby leaving a 583 amino acid sequence. Accordingly, the compound, which causes, or contributes to, neurodegeneration of the brain slices, comprises or consists of an amino acid sequence derived from the C-terminus of acetylcholinesterase, or a truncation thereof, wherein the acetylcholinesterase comprises an amino acid sequence substantially as set out in SEQ ID No:1, preferably excluding the 31 amino acids at the N-terminal.

Preferably, the compound comprises or consists of an amino acid sequence derived from the last 300, 200, 100 or 50 amino acids forming the C-terminus of acetylcholinesterase, or a truncation thereof, preferably wherein the acetylcholinesterase comprises an amino acid sequence substantially as set out in SEQ ID No:1. The compound preferably comprises or consists of a polypeptide, derivative or analogue thereof which comprises an amino acid sequence derived from the last 40 amino acids forming the C-terminus of acetylcholinesterase, or a truncation thereof.

Preferably, the compound comprises or consists of a polypeptide, derivative or analogue thereof of between 8 and 40 amino acid residues, more preferably between 10 and 35 amino acids, and most preferably between 12 and 20 amino acids. The inventor has prepared a sequence alignment between β-amyloid (Aβ), and three peptides that are derived from the C-terminus of AChE, which are referred to herein as T30, T14 and T15.

The amino acid sequence of part of β-amyloid (Aβ) is provided herein as SEQ ID No:2, as follows:-
DAEFRHDSGYEVHHQKLVFFAEDVGSNKGAIIGLMVGGVVIA
[SEQ ID No:2]

The amino acid sequence of T30 (which corresponds to the last 30 amino acid residues of SEQ ID No:1) is provided herein as SEQ ID No:3, as follows:-
KAEFHRWSSYMVHWKNQFDHYSKQDRCSDL
[SEQ ID No:3]

The amino acid sequence of T14 (which corresponds to the 14 amino acid residues located towards the end of SEQ ID No:1, and lacks the final 15 amino acids found in T30) is provided herein as SEQ ID No:4, as follows:-
AEFHRWSSYMVHWK
[SEQ ID No:4]

The amino acid sequence of T15 (which corresponds to the last 15 amino acid residues of SEQ ID No:1) is provided herein as SEQ ID No:5, as follows:-
NQFDHYSKQDRCSDL
[SEQ ID No:5]

Accordingly, preferably the compound, which contributes to, or causes, neurodegeneration of the brain section comprises or consists of SEQ ID No: 3, 4 or 5. Most preferably, as illustrated in the Examples, the compound is T30 (SEQ ID No:3).

The term "derivative or analogue thereof' can mean a polypeptide within which amino acid residues are replaced by residues (whether natural amino acids, non-natural amino acids or amino acid mimics) with similar side chains or peptide backbone properties. Additionally, the terminals of such peptides may be protected by N- and C-terminal protecting groups with similar properties to acetyl or amide groups.

Derivatives and analogues of polypeptides according to the invention may also include those that increase the peptide's half-life in vivo. For example, a derivative or analogue of the peptides of the invention may include peptoid and retropeptoid derivatives of the peptides, peptide-peptoid hybrids and D-amino acid derivatives of the peptides.

Peptoids, or poly-N-substituted glycines, are a class of peptidomimetics whose side chains are appended to the nitrogen atom of the peptide backbone, rather than to the alpha-carbons, as they are in amino acids. Peptoid derivatives of the peptides of the invention may be readily designed from knowledge of the structure of the peptide. Retropeptoids (in which all amino acids are replaced by peptoid residues in reversed order) are also suitable derivatives in accordance with the invention. A retropeptoid is expected to bind in the opposite direction in the ligand-binding groove, as compared to a peptide or peptoid-peptide hybrid containing one peptoid residue. As a result, the side chains of the peptoid residues are able point in the same direction as the side chains in the original peptide.

In another preferred embodiment, the compound, which contributes to, or causes, neurodegeneration of the brain section, is a peptidomimetic compound having Formula (I): 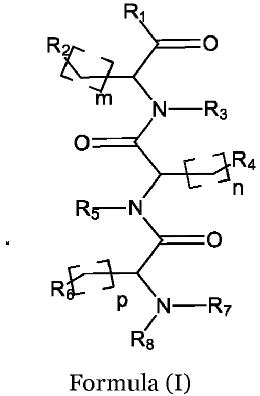 , wherein:
R₁ is -NR₉R₁₀ or -OH;
R₂ is -NH₂, -OH, -N(H)C(NH)NH₂, -C(O)OH or -C(O)NH₂;
R₃ is -H or a C₁₋₅ straight or branched alkyl or alkenyl; or
R₂ and R₃ together with the nitrogen and carbon(s) to which they are bonded form a ring substituted by -OH or -NH2;
R₄ is aryl or heteroaryl, optionally substituted with one or more X₁ groups;
R₅ is -H or a C₁₋₅ straight or branched alkyl or alkenyl;
R₆ is aryl or heteroaryl, optionally substituted with one or more X₁ groups;
R₇ is -H or a C₁₋₅ straight or branched alkyl or alkenyl;
R₈ is -H, a C₁₋₅ straight or branched alkyl or alkenyl or -C(O)R₁₂;
the or each X₁ group is independently selected from -NR₉R₁₀, - OH or -C(O)R₁₂;
the or each R₉ and R₁₀ are independently -H or a C₁₋₅ straight or branched alkyl or alkenyl;
R₁₁ is -NH2, -OH or an aryl group;
R₁₂ is a C₁₋₅ straight or branched alkyl or alkenyl; and
m, n and p are each independently between 0 and 10;
or a salt, solvate, tautomeric form, or polymorphic form thereof.

It will be appreciated that, in some embodiments, the incubation media in at least one of the containers may comprise more than one compound which contributes to, or causes, neurodegeneration of the brain section contained therein. Similarly, in some embodiments the method may comprise contacting at least one of the brain sections with more than one compound which contributes to, or causes, neurodegeneration of the brain section. The more than one compound may comprise a polypeptide, derivative or analogue thereof and a peptidomimetic compound, wherein the polypeptide, derivative or analogue thereof and the peptidomimetic compound are as defined herein.

The inventors believe that this compound is novel *per se.*

Hence, in a sixth aspect, there is provided a compound of Formula (I): 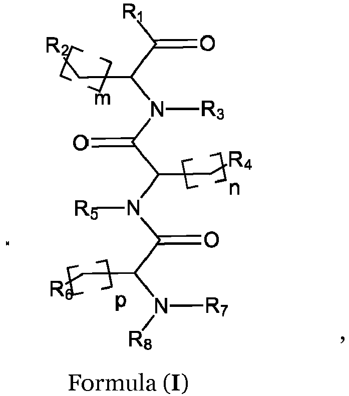 wherein:
R₁ is -NR₉R₁₀ or -OH;
R₂ is -NH₂, -OH, -N(H)C(NH)NH₂, -C(O)OH or -C(O)NH₂;
R₃ is -H or a C₁₋₅ straight or branched alkyl or alkenyl; or
R₂ and R₃ together with the nitrogen and carbon(s) to which they are bonded form a ring substituted by -OH or -NH2;
R₄ is aryl or heteroaryl, optionally substituted with one or more X₁ groups;
R₅ is -H or a C₁₋₅ straight or branched alkyl or alkenyl;
R₆ is aryl or heteroaryl, optionally substituted with one or more X₁ groups;
R₇ is -H or a C₁₋₅ straight or branched alkyl or alkenyl;
R₈ is -H, a C₁₋₅ straight or branched alkyl or alkenyl or -C(O)R₁₂;
the or each X₁ group is independently selected from -NR₉R₁₀, - OH or -C(O)R₁₂;
the or each R₉ and R₁₀ are independently -H or a C₁₋₅ straight or branched alkyl or alkenyl;
R₁₁ is -NH2, -OH or an aryl group;
R₁₂ is a C₁₋₅ straight or branched alkyl or alkenyl; and
m, n and p are each independently between 0 and 10;
or a salt, solvate, tautomeric form, or polymorphic form thereof.

It may be understood that the term "salt" refers to any salt of a compound provided herein which retains its biological properties. Such salts may be derived from a variety of organic and inorganic counter-ions well known in the art. Such salts include, but are not limited to: (1) acid addition salts formed with organic or inorganic acids such as hydrochloric, hydrobromic, sulfuric, nitric, phosphoric, sulfamic, acetic, trifluoroacetic, trichloroacetic, propionic, hexanoic, cyclopentylpropionic, glycolic, glutaric, pyruvic, lactic, malonic, succinic, sorbic, ascorbic, malic, maleic, fumaric, tartaric, citric, benzoic, 3-(4-hydroxybenzoyl)benzoic, picric, cinnamic, mandelic, phthalic, lauric, methanesulfonic, ethanesulfonic, 1,2-ethane-disulfonic, 2-hydroxyethanesulfonic, benzenesulfonic, 4-chlorobenzenesulfonic, 2-naphthalenesulfonic, 4-toluenesulfonic, camphoric, camphorsulfonic, 4-methylbicyclo[2.2.2]-oct-2-ene-1-carboxylic, glucoheptonic, 3-phenylpropionic, trimethylacetic, tert-butylacetic, lauryl sulfuric, gluconic, benzoic, glutamic, hydroxynaphthoic, salicylic, stearic, cyclohexylsulfamic, quinic, muconic acid and the like acids; or (2) base addition salts formed when an acidic proton present in the parent compound either (a) is replaced by a metal ion, e.g., an alkali metal ion, an alkaline earth ion or an aluminium ion, or alkali metal or alkaline earth metal hydroxides, such as sodium, potassium, calcium, magnesium, aluminium, lithium, zinc, and barium hydroxide, ammonia or (b) coordinates with an organic base, such as aliphatic, alicyclic, or aromatic organic amines, such as ammonia, methylamine, dimethylamine, diethylamine, picoline, ethanolamine, diethanolamine, triethanolamine, ethylenediamine, lysine, arginine, ornithine, choline, N,N'-dibenzylethylene-diamine, chloroprocaine, diethanolamine, procaine, N-benzylphenethylamine, N-methylglucamine piperazine, tris(hydroxymethyl)-aminomethane, tetramethylammonium hydroxide, and the like.

Salts may further include, by way of example only and without limitation, sodium, potassium, calcium, magnesium, ammonium, tetraalkylammonium and the like, and when the compound contains a basic functionality, salts of organic or inorganic acids, such as hydrohalides, e.g. hydrochloride and hydrobromide, sulfate, phosphate, sulfamate, nitrate, acetate, trifluoroacetate, trichloroacetate, propionate, hexanoate, cyclopentylpropionate, glycolate, glutarate, pyruvate, lactate, malonate, succinate, sorbate, ascorbate, malate, maleate, fumarate, tartarate, citrate, benzoate, 3-(4-hydroxybenzoyl)benzoate, picrate, cinnamate, mandelate, phthalate, laurate, methanesulfonate (mesylate), ethanesulfonate, 1,2-ethane-disulfonate, 2-hydroxyethanesulfonate, benzenesulfonate (besylate), 4-chlorobenzenesulfonate, 2-naphthalenesulfonate, 4-toluenesulfonate, camphorate, camphorsulfonate, 4-methylbicyclo[2.2.2]-oct-2-ene-1-carboxylate, glucoheptonate, 3-phenylpropionate, trimethylacetate, tert-butylacetate, lauryl sulfate, gluconate, benzoate, glutamate, hydroxynaphthoate, salicylate, stearate, cyclohexylsulfamate, quinate, muconate and the like.

It may be understood that the term "solvate" refers to a compound provided herein or a salt thereof, that further includes a stoichiometric or non-stoichiometric amount of solvent bound by non-covalent intermolecular forces. Where the solvent is water, the solvate is a hydrate.

It may be appreciated that an aryl group refers to a substituent derived from an aromatic ring. The aryl group may be a C₆-C₁₂ aryl group. Preferably, the aryl group is phenyl, biphenyl or naphthyl.

It may be appreciated that a heteroaryl group refers to refers to a monovalent monocyclic aromatic group and/or multicyclic aromatic group that contain at least one aromatic ring, wherein at least one aromatic ring contains one or more heteroatoms independently selected from O, S and N in the ring. Heteroaryl groups are bonded to the rest of the molecule through the aromatic ring. Each ring of a heteroaryl group can contain one or two O atoms, one or two S atoms, and/or one to four N atoms, provided that the total number of heteroatoms in each ring is four or less and each ring contains at least one carbon atom. In certain embodiments, the heteroaryl has from 5 to 20, from 5 to 15, or from 5 to 10 ring atoms. Examples of monocyclic heteroaryl groups include, but are not limited to, furanyl, imidazolyl, isothiazolyl, isoxazolyl, oxadiazolyl, oxadiazolyl, oxazolyl, pyrazinyl, pyrazolyl, pyridazinyl, pyridyl, pyrimidinyl, pyrrolyl, thiadiazolyl, thiazolyl, thienyl, tetrazolyl, triazinyl and triazolyl. Examples of bicyclic heteroaryl groups include, but are not limited to, benzofuranyl, benzimidazolyl, benzoisoxazolyl, benzopyranyl, benzothiadiazolyl, benzothiazolyl, benzothienyl, benzotriazolyl, benzoxazolyl, furopyridyl, imidazopyridinyl, imidazothiazolyl, indolizinyl, indolyl, indazolyl, isobenzofuranyl, isobenzothienyl, isoindolyl, isoquinolinyl, isothiazolyl, naphthyridinyl, oxazolopyridinyl, phthalazinyl, pteridinyl, purinyl, pyridopyridyl, pyrrolopyridyl, quinolinyl, quinoxalinyl, quinazolinyl, thiadiazolopyrimidyl, and thienopyridyl. Examples of tricyclic heteroaryl groups include, but are not limited to, acridinyl, benzindolyl, carbazolyl, dibenzofuranyl, perimidinyl, phenanthrolinyl, phenanthridinyl, phenarsazinyl, phenazinyl, phenothiazinyl, phenoxazinyl and xanthenyl. In certain embodiments, heteroaryl may also be optionally substituted as described herein.

Most preferably, the compound has Formula (Ia): 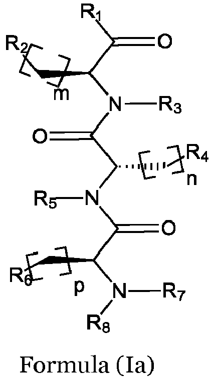 R₁ may be -OH. However, preferably, R₁ is -NR₉R₁₀, more preferably R₁ is -NR₉H, and most preferably R₁ is -NH2.

Preferably, R₂ and R₃ together with the nitrogen and carbon to which they are bonded form a ring substituted by -OH or -NH2. Accordingly, the compound may have Formula (Ib): 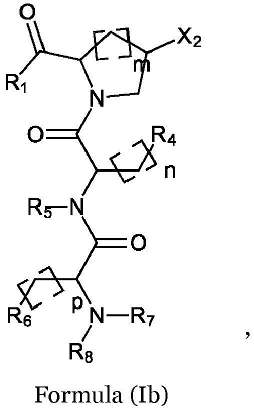 wherein, X₂ is -OH or -NH₂.

More preferably, the compound has Formula (Ic):

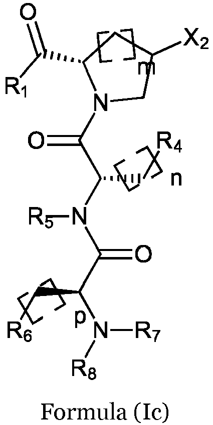

Most preferably, the compound has Formula (Id):

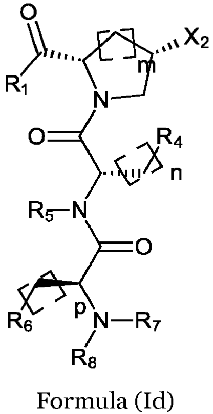

Preferably, m is 0 to 5. Accordingly, m may be 0, 1, 2, 3, 4 or 5, and most preferably m is 1.

In a preferred embodiment, X₂ is -OH.

Preferably, R₄ is a C₆-C₁₂ aryl group or a heteroaryl group having from 5 to 20 ring atoms, substituted with one or more X₁ groups. More preferably, R₄ is a C₆-C₁₂ aryl group substituted with one or more X₁ groups. Most preferably, R₄ is a phenyl group substituted with one or more X₁ groups.

Preferably, R₄ is substituted with one X₁ group. Preferably, R₄ is a phenyl group and X₁ is disposed in the para position.

Preferably, X₁ is -C(O)R₁₁, and more preferably -C(O)OH.

Accordingly, in a preferred embodiment, R₄ is

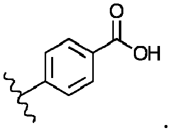

Preferably, n is 0 to 5. Accordingly, n may be 0, 1, 2, 3, 4 or 5, and most preferably n is 1.

It will be appreciated that R₅ may be a methyl, ethyl, propyl, butyl or pentyl group. Preferably, R₅ is methyl. However, in a more preferred embodiment, R₅ is -H.

Preferably, R₆ is a C₆-C₁₂ aryl group or a heteroaryl group having from 5 to 20 ring atoms. Preferably, R₆ is a bicyclic aryl group or bicyclic heteroaryl group. Preferably, R₆ is an aryl group, and more preferably, a bicyclic aryl group. Most preferably, R₆ is a naphthyl group.

Preferably, p is 0 to 5. Accordingly, p may be 0, 1, 2, 3, 4 or 5, and most preferably p is 1.

It will be appreciated that R₇ may be a methyl, ethyl, propyl, butyl or pentyl group. Preferably, R₇ is methyl. However, in a more preferred embodiment, R₇ is -H.

In one preferred embodiment R₈ is -H. However, in a more preferred embodiment R₈ is -C(O)R₁₂. It will be appreciated that R₁₂ may be a methyl, ethyl, propyl, butyl or pentyl group. Preferably, R₁₂ is methyl.

In a preferred embodiment, the compound has Formula (Ie):

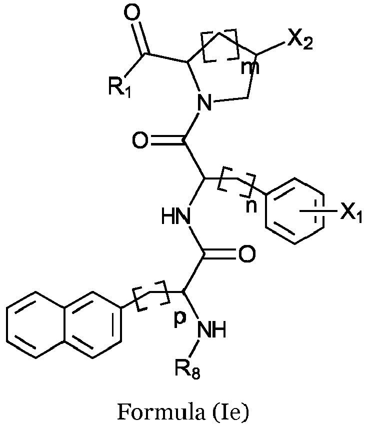

More preferably, the compound has Formula (If):

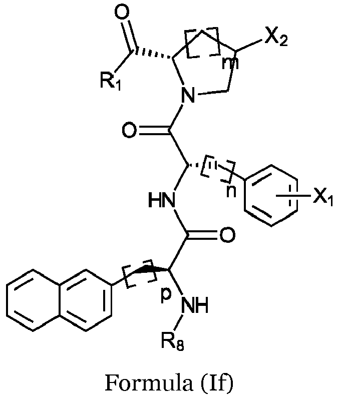

Most preferably, the compound has Formula (Ig):

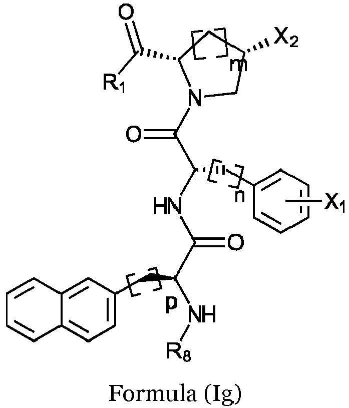

In an alternative preferred embodiment, the compound has Formula (Ih):

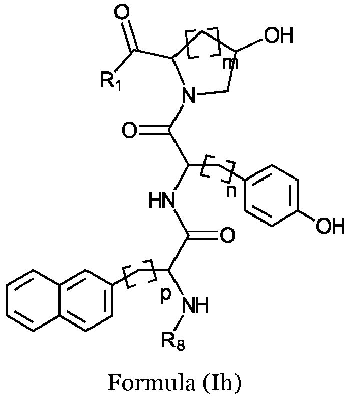

More preferably, the compound has Formula (Ii):

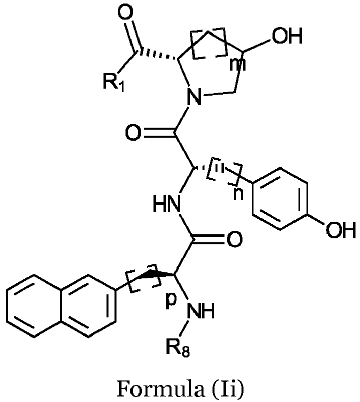

Most preferably, the compound has Formula (Ij):

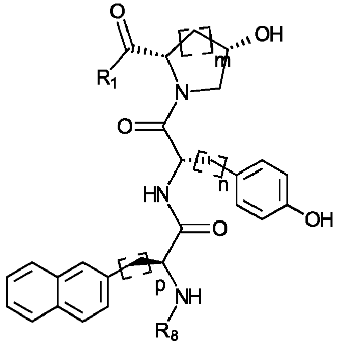

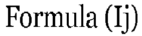

In a further alternative preferred embodiment, the compound has Formula (Ik):

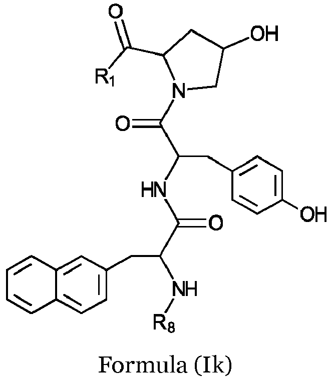

Preferably, the compound has Formula (Il):

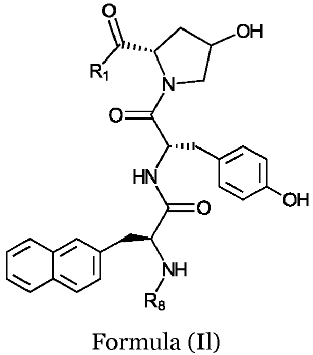

Most preferably, the compound has Formula (Im):

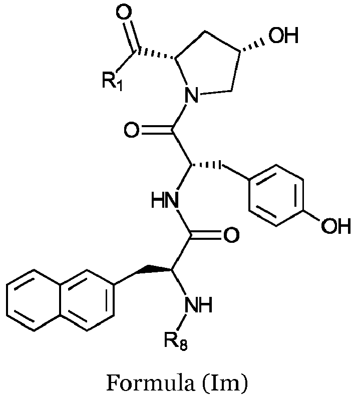

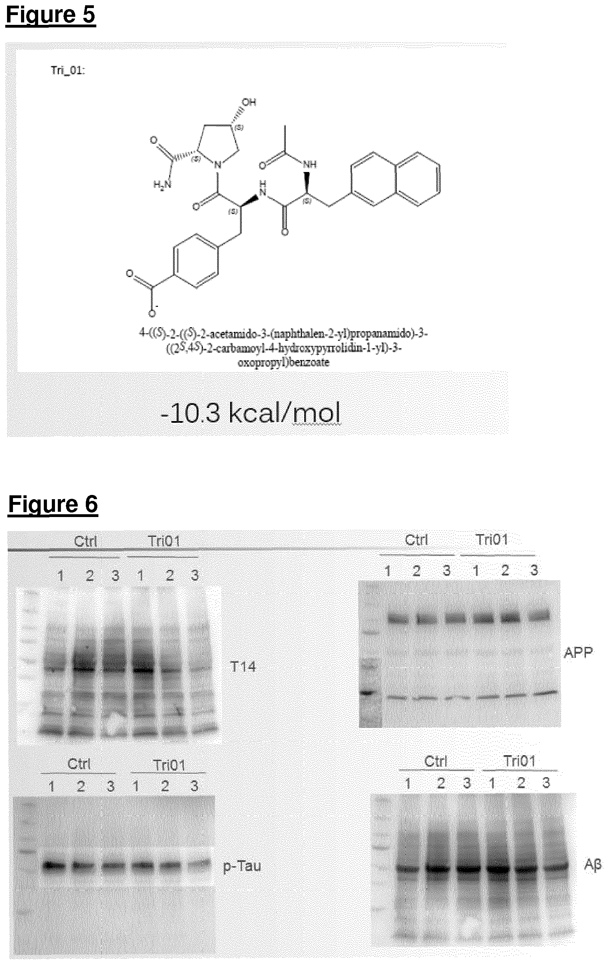

Preferably, R₁ is -NH2 and R₈ is -C(O)CH₃. Hence, a most preferred compound which causes, or contributes to, neurodegeneration in the brain section is compound of Formula Im, in which R₁ is -NH₂ and R₈ is -C(O)CH₃, known as Tri01, as shown in Figure 5.

Preferably, the compound which contributes to, or causes, neurodegeneration of the brain section described herein, is as defined in accordance with the sixth aspect.

It will be appreciated that the focus of the invention is on the investigation of neurodegeneration disorders, such as Alzheimer's. However, in addition to investigating neurodegenerative disorders, the inventors believe that the apparatus and methods of the invention can also be used to study neuroregeneration. For example, contacting the brain slices with one or more trophic factors enables the evaluation of how the brain slices react to the treatment, i.e. investigating the synthesis of endogenous trophic factors, axonal and dendritic rearrangement (reorganization).

Thus, in a seventh aspect of the invention, there is provided an apparatus for carrying out an animal model for neuroregeneration, the apparatus comprising:-
- a first container configured to receive a first brain section from an animal brain, and comprising incubation media for retaining viability of the brain section;
- a second container configured to receive a second brain section, which is contralateral with the first brain section, and comprising incubation media for retaining viability of the brain section; and
- oxygen feed means configured to feed oxygen into the media in the first and second containers to thereby incubate the first and second brain sections to retain their viability,
wherein the incubation media in at least one of the containers comprises a compound which contributes to, or causes, neuroregeneration of the brain section contained therein.

The inventors have also devised a method for producing an animal model for neuroregeneration.

Hence, in an eighth aspect, there is provided a method of providing an animal model for neuroregeneration, the method comprising:-
- removing a region of an animal brain, and dividing the brain region into corresponding first and second contralateral brain sections;
- incubating the first and second brain sections to retain their viability; and
- contacting at least one of the brain sections with a compound which contributes to, or causes, neuroregeneration of the brain section.

The apparatus of the seventh aspect and method of the eighth aspect preferably comprise features from the first to sixth aspects.

Preferably, the brain section in the test container is exposed to an effective amount of the compound such that it contributes to, or causes, neuroregeneration. For example, the concentration of the compound may be between 0.1 µM and 1000µM, or between 0.5 µM and 500µM, or between 1µM and 100µM, or between 0.15 µM and 10µM. Preferably, the brain section in the test container is exposed to the compound, which contributes to, or causes, neuroregeneration for at least 1 hour, 2 hours, 3 hours, 4 hours, or 5 hours. Preferably, the brain section in the test container is exposed to the compound for less than 7 hours or 6 hours. Most preferably, the brain section in the test container is exposed to the compound for between 1 and 7 hours, even more preferably between 2 and 6 hours, and most preferably between 3 and 5.5 hours. In a most preferred embodiment, a maximum exposure time of about 5 hours is preferred.

Preferably, the compound which contributes to, or causes, neuroregeneration of the brain section is a trophic factor. Examples of suitable compounds include nerve growth factor (NGF), brain derived neurotrophic factor (BDNF) and fibroblast growth factors (FGFs). Another trophic factor may be T14 peptide, which is known to have both trophic as well as toxic effects.

It will be appreciated that the invention extends to any nucleic acid or peptide or variant, derivative or analogue thereof, which comprises substantially the amino acid or nucleic acid sequences of any of the sequences referred to herein, including functional variants or functional fragments thereof. The terms "substantially the amino acid/nucleotide/peptide sequence", "functional variant" and "functional fragment", can be a sequence that has at least 40% sequence identity with the amino acid/nucleotide/peptide sequences of any one of the sequences referred to herein, for example 40% identity with the sequence identified herein.

Amino acid/polynucleotide/polypeptide sequences with a sequence identity which is greater than 65%, more preferably greater than 70%, even more preferably greater than 75%, and still more preferably greater than 80% sequence identity to any of the sequences referred to are also envisaged. Preferably, the amino acid/polynucleotide/polypeptide sequence has at least 85% identity with any of the sequences referred to, more preferably at least 90% identity, even more preferably at least 92% identity, even more preferably at least 95% identity, even more preferably at least 97% identity, even more preferably at least 98% identity and, most preferably at least 99% identity with any of the sequences referred to herein, i.e. SEQ ID No:1-5.

The skilled technician will appreciate how to calculate the percentage identity between two amino acid/polynucleotide/polypeptide sequences. In order to calculate the percentage identity between two amino acid/polynucleotide/polypeptide sequences, an alignment of the two sequences must first be prepared, followed by calculation of the sequence identity value. The percentage identity for two sequences may take different values depending on:- (i) the method used to align the sequences, for example, ClustalW, BLAST, FASTA, Smith-Waterman (implemented in different programs), or structural alignment from 3D comparison; and (ii) the parameters used by the alignment method, for example, local vs global alignment, the pair-score matrix used (e.g. BLOSUM62, PAM250, Gonnet etc.), and gap-penalty, e.g. functional form and constants.

Having made the alignment, there are many different ways of calculating percentage identity between the two sequences. For example, one may divide the number of identities by: (i) the length of shortest sequence; (ii) the length of alignment; (iii) the mean length of sequence; (iv) the number of non-gap positions; or (v) the number of equivalenced positions excluding overhangs. Furthermore, it will be appreciated that percentage identity is also strongly length dependent. Therefore, the shorter a pair of sequences is, the higher the sequence identity one may expect to occur by chance.

Hence, it will be appreciated that the accurate alignment of protein or DNA sequences is a complex process. The popular multiple alignment program ClustalW (Thompson et al., 1994, Nucleic Acids Research, 22,4673-4680; Thompson et al., 1997, Nucleic Acids Research, 24, 4876-4882) is a preferred way for generating multiple alignments of proteins or DNA in accordance with the invention. Suitable parameters for ClustalW may be as follows: For DNA alignments: Gap Open Penalty = 15.0, Gap Extension Penalty = 6.66, and Matrix = Identity. For protein alignments: Gap Open Penalty = 10.0, Gap Extension Penalty = 0.2, and Matrix = Gonnet. For DNA and Protein alignments: ENDGAP = -1, and GAPDIST = 4. Those skilled in the art will be aware that it may be necessary to vary these and other parameters for optimal sequence alignment.

Preferably, calculation of percentage identities between two amino acid/polynucleotide/polypeptide sequences may then be calculated from such an alignment as (N/T)*100, where N is the number of positions at which the sequences share an identical residue, and T is the total number of positions compared including gaps but excluding overhangs. Hence, a most preferred method for calculating percentage identity between two sequences comprises (i) preparing a sequence alignment using the ClustalW program using a suitable set of parameters, for example, as set out above; and (ii) inserting the values of N and T into the following formula:-Sequence Identity = (N/T)*100.

Alternative methods for identifying similar sequences will be known to those skilled in the art. For example, a substantially similar nucleotide sequence will be encoded by a sequence, which hybridizes to DNA sequences or their complements under stringent conditions. By stringent conditions, we mean the nucleotide hybridises to filter-bound DNA or RNA in 3x sodium chloride/sodium citrate (SSC) at approximately 45°C followed by at least one wash in 0.2x SSC/0.1% SDS at approximately 20-65°C. Alternatively, a substantially similar polypeptide may differ by at least 1, but less than 5, 10, 20, 50 or 100 amino acids from the sequences shown in SEQ ID No: 1-5.

Due to the degeneracy of the genetic code, it is clear that any nucleic acid sequence described herein could be varied or changed without substantially affecting the sequence of the protein encoded thereby, to provide a functional variant thereof. Suitable nucleotide variants are those having a sequence altered by the substitution of different codons that encode the same amino acid within the sequence, thus producing a silent change. Other suitable variants are those having homologous nucleotide sequences but comprising all, or portions of, sequence, which are altered by the substitution of different codons that encode an amino acid with a side chain of similar biophysical properties to the amino acid it substitutes, to produce a conservative change. For example small non-polar, hydrophobic amino acids include glycine, alanine, leucine, isoleucine, valine, proline, and methionine. Large non-polar, hydrophobic amino acids include phenylalanine, tryptophan and tyrosine. The polar neutral amino acids include serine, threonine, cysteine, asparagine and glutamine. The positively charged (basic) amino acids include lysine, arginine and histidine. The negatively charged (acidic) amino acids include aspartic acid and glutamic acid. It will therefore be appreciated which amino acids may be replaced with an amino acid having similar biophysical properties, and the skilled technician will know the nucleotide sequences encoding these amino acids.

All of the features described herein (including any accompanying claims, abstract and drawings), and/or all of the steps of any method or process so disclosed, may be combined with any of the above aspects in any combination, except combinations where at least some of such features and/or steps are mutually exclusive.

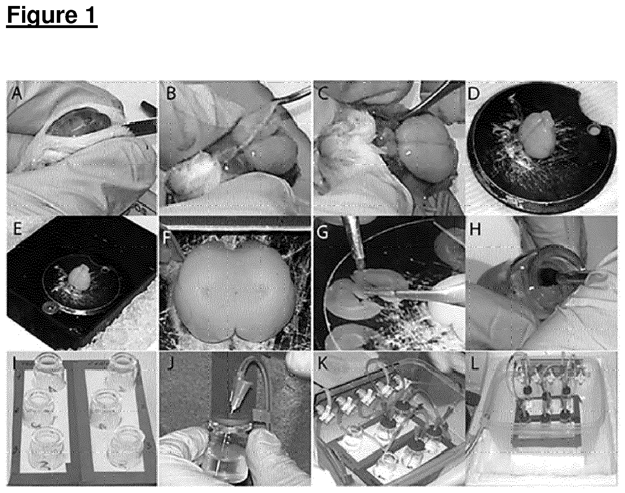

For a better understanding of the invention, and to show how embodiments of the same may be carried into effect, reference will now be made, by way of example, to the accompanying Figures, in which:-
**Figure 1** shows step-by-step visualisation of the method and apparatus of the invention, for conducting an ex vivo brain slice assay. (A-C) Dissection of the brain from the skull. (D) Attachment of the brain to the vibratome disc. (E) The outer buffer-chamber is ice cooled, the inner part contains the brain and is filled with ice cold slicing artificial cerebrospinal fluid (aCSF). (F) Sectioning of the brain in coronal slices containing the basal forebrain (BF). (G) Division of the slices in two complementary hemisections. (H) Disposal of each hemisection in its corresponding vial. (I) Three hemisections are transferred into the control group vials (green label) which include, from top to the bottom, the rostral, the intermediate and the caudal region of the basal forebrain. The same order is maintained for the treated group (red label). (J-K) Each vial is individually sealed, oxygen provided (J) and placed into the support (K). (L) Set up ready for the experiment, connected to the oxygen source and placed on ice;
**Figure 2** shows a schematic side view of an apparatus of the invention for carrying out the ex vivo assay;

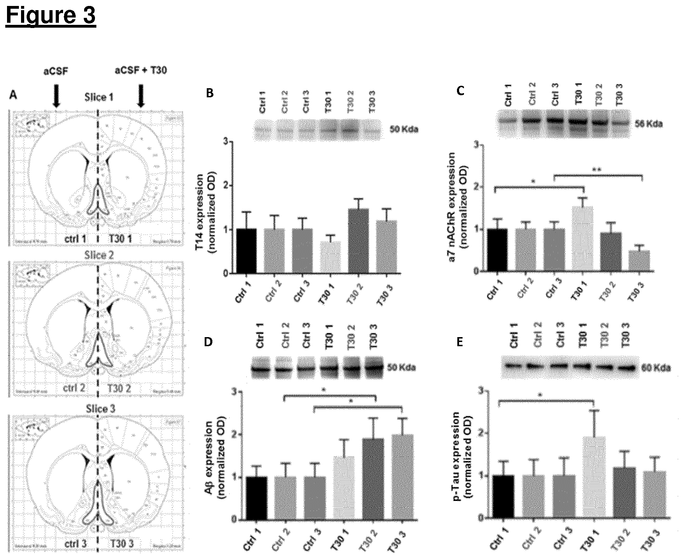

**Figure 3** shows that T30 induces a differential expression of α7-nAchR, p-Tau and Aβ. (A) Brain atlas coronal slices containing BF (in red), obtained at three anatomical planes of sectioning. (B-E) Representative western blot analysis and related bar graphs indicating the levels of endogenous T14 (B), α7-nAChR (C), Aβ (C) and p-Tau (D). (B) AChE peptide effect does not induce any significant difference in T14 levels along the rostro-caudal axis, when comparing the control and treated group. (C) T30 administration triggers two opposite effects on the α7-nAChR expression, inducing an increase in the rostral section (T30 1) and a reduction in the caudal region (T30 3) compared to the control counterparts (ctrl 1 and ctrl 3), whereas there is no difference in the intermediate slice (ctrl 2, T30 2). (D) Amyloid beta expression is significantly increased in the intermediate (T30 2) and posterior (T30 3) treated hemisection compared to their corresponding control sides (ctrl 2 and ctrl 3). (E) p-Tau levels are enhanced in the anterior portion (T30 1) compared to the control matching hemisection (ctrl 1) while no changes between treated and untreated condition are observed in the other two sections (slice 2 and 3). * p < 0.05, ** p < 0.01. Error bars indicate SEM;

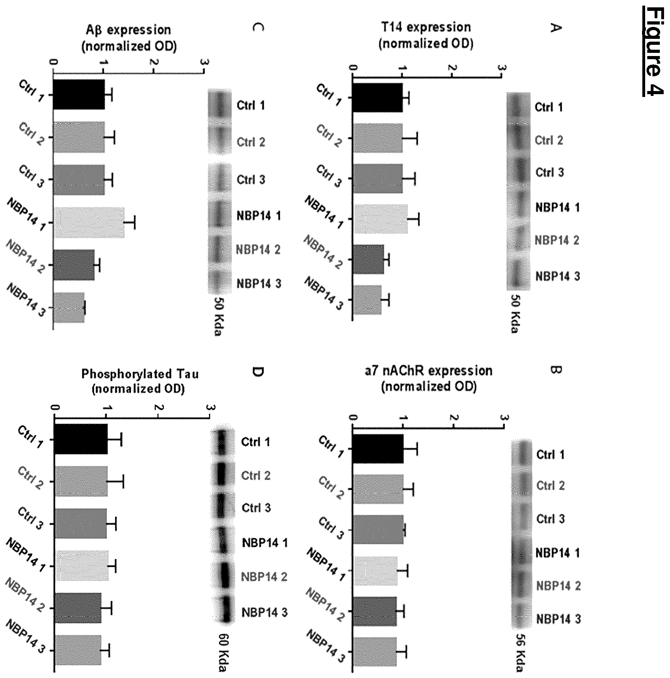

**Figure 4** shows representative WB analysis and related graphs indicating the levels of endogenous T14, α7-nAChR, Aβ and p-Tau along the rostro-caudal axis upon NBP14 exposure. **(A)** T14 showed a continuous reduction in the treated hemisections, but no difference was observed when compared to their control matching counterparts. **(B)** NBP14 administration did not affect the α7-nAChR expression, which is similar to the control group. **(C)** Aβ expression was progressively decreased in the treated group, but no difference was observed when compared to the untreated one. **(D)** p-Tau levels were unaffected after NBP14 application, showing no changes when compared to the control condition. A group of 5 animals was used for these experiments. Error bars indicate SEM.
**Figure 5** shows the structure of a peptidomimetic compound, Tri01;
**Figure 6** shows the Western blot results of the administration of the Tri01 peptidomimetic in the *ex-vivo* animal model, i.e. its effects on the expression of T14, p-Tau, APP and A-beta;
**Figure 7** shows the effects of Tri01 (with and without T30) on PC12 cell viability;
**Figure 8** shows the results of the AChE activity test following Tri01 administration to PC12 cells (with and without T30); and
**Figure 9** shows the results of the Calcium influx test following Tri01 administration to PC12 cells (with and without T30).

### Examples

Despite the increasing numbers of studies targeting the primary events in neurodegeneration, there is no animal model which closely reproduces the full pathological profile (e.g. of Alzheimer's disease), since the basic mechanisms of neurodegeneration are still poorly understood. Thus, the inventors have developed a novel apparatus and animal model to elucidate the basic mechanisms inducing neurodegeneration, and, importantly, in which novel test agents could be tested to determine neuroprotective (or neurotoxic) activity. The invention involves the use of a peptide cleaved from the C-terminus of acetylcholinesterase (AChE), T30 (SEQ ID NO:3), which is composed by a bioactive portion, T14 (SEQ ID No: 4), and an inert fragment, T15 (SEQ ID No: 5) that interacts with the α7 nicotinic acetylcholine receptor (α7-nAChR). The inventors have previously shown that the application of AChE-derived peptide on cell lines promotes an AD-like phenotype. These effects are blocked by a novel candidate modulator of the α7-nAChR, NBP-14, which is the cyclized form of T14, and so has the sequence of cyclic SEQ ID No:4. As described below, the inventors have applied T30 or NBP14 on ex vivo brain slices and investigated their activity in modulating the endogenous T14 expression, and whether they contribute or prevent a neurodegeneration pattern. In addition to studying the effects of T30 to induce or replicate neurodegenerative damage, and the effects of NBP14 which prevents neurodegeneration, the inventors have also created a novel peptidomimetic compound referred to as "Tri 01" based on the structure of NBP14, which can be used in the model and *ex-vivo* assay apparatus.

The inventors show that the apparatus and model can be used to study neurodegeneration in a more physiological scenario, i.e. *ex vivo* brain slices, on α7-nAChR, p-Tau and Aβ expression, though it will be appreciated that there any many other proteins that can be measured to monitor degree and progression of neurodegenerative disorders. The model harnesses the inventors' new hypothesis which they believe accounts for the aberrant processes characterizing AD, based on the interaction between the α7 nicotinic acetylcholine receptor (α7-nAChR) and the toxic peptide, cleaved from the acetylcholinesterase (AChE) C-terminus, i.e. T30. The apparatus and models can be used to examine many molecular processes, test pharmacological compounds which may regulate these processes and provide a reliable tool for drug screening, reducing whole animal experiments.

### Example 1 - Use of T30 in ex-vivo assay/model

### Materials and Methods

### Animals

Five wild type P14 male Wistar rats were sacrificed as previously described [15]. The use of animals in this study was in accordance with the UK (ASPA regulations) and European guidelines on animal experimentation.

### Reagents for artificial cerebrospinal fluids (αCSFs) preparation

Sodium chloride (NaCl; Sigma-Aldrich, S7653, Germany); Potassium chloride (KCl; Sigma-Aldrich, P9333, Germany); Sodium bicarbonate (NaHCO₃; Sigma-Aldrich, S5761, Germany); Magnesium sulphate heptahydrate (MgSO₄(7H₂O); Sigma-Aldrich, 63138, Germany); Potassium phosphate monobasic (KH₂PO₄; Sigma-Aldrich, P5655, Germany); Hepes salt (Sigma-Aldrich, H7006, Germany); Hepes acid (Sigma-Aldrich, H3375, Germany); Glucose (Sigma-Aldrich, G7528, Germany); Calcium chloride dehydrate (Sigma-Aldrich, 223506, Germany); T30 (Genosphere Biotechnologies, France).

These reagents are used to obtain the stock solutions 5x Krebs, 5x Hepes and Sodium bicarbonate in order to prepare the "slicing" aCSF and the "recording" aCSF.

The final concentrations of the "slicing" and "recording" aCSF has been previously described [15].

Briefly, "slicing" aCSF in mmol: 120 NaCl, 5 KCl, 20 NaHCO₃, 2.4 CaCl2, 2 MgSO₄, 1.2 KH2PO₄ and 10 glucose; 6.7 HEPES salt and 3.3 HEPES acid; pH: 7.1. "Recording" aCSF in mmol: 124 NaCl, 3.7 KCl, 26 NaHCO₃, 2 CaCl2, 1.3 MgSO₄, 1.3 KH2PO₄ and 10 glucose; pH: 7.1.

### Equipment and reagents for brain dissection, slicing, incubation and homogenization

Plastic box; Isoflurane (Henry Schein, cat # 200-070, USA); Guillotine; aCSFs; Surgical dissecting kit (World Precision Instruments, Item #: MOUSEKIT, USA); Surgical blades (Swann-Morton, BS 2982, UK); Spatula; Glue; Filter paper (Fisher, cat #11566873, USA); Vibratome (Leica, VT1000 S, Germany); Brushes; Icebox; Oxygen canister; Apparatus (see materials and instructions below); 1x Phosphate buffer saline (PBS, Fisher, cat # BP2438-4, USA); Phosphatase inhibitors (Fisher, cat # 1284-1650, USA); Protease inhibitors (Roche complete PIC, 04693116001, USA); Pestles (Starlab, cat# I1415-5390, UK); Microcentrifuge tubes 1.5-2 ml.

### Western blot reagents and equipment

Pierce 660 nm Protein Assay (Thermo Scientific, cat # 22660, USA); Loading buffer (Bio Rad, cat # 161-0747, USA); Beta-mercaptoethanol (Bio Rad, cat # 161-0710, USA) Ladder (Bio Rad, cat # 161-0377, USA); Running buffer 10x TGS (Fisher, BP1341, cat # 10102823, USA); Transfer Buffer 10x, prepared dissolving Tris Base (Fisher Scientific, BP152-500, USA) and Glycine (Fisher Scientific, BP381-500, USA) in distilled water; Tris Buffer Saline, TBS 10x prepared dissolving Tris Base (Fisher Scientific, BP152-500, USA) and sodium chloride (Sigma-Aldrich, S7653, Germany) in distilled water; Tween 20 (Sigma-Aldrich, P9416, Germany); Filter paper (Bio Rad, cat # 1703955, USA); PVDF (Immobilon-P) transfer membranes (Sigma-Aldrich, P2563, Germany); 4-20% Mini-PROTEAN TGX Precast Protein Gels (Bio Rad, cat # 456-1094, USA); BLOT-FastStain (G-Biosciences, cat # 786-34, USA); Blotting grade blocker (Bio Rad, cat # 170-6404, USA); Mouse anti-phosphorylated Tau (Thermo Fisher, MN1020, UK); Mouse anti-Amyloid beta (Absolute Antibody, Ab00239-2.0, UK); Rabbit anti-α7-nAchR (Abcam, ab10096, UK); Goat anti mouse HRP conjugated (Sigma-Aldrich, A9309, Germany); Goat anti rabbit HRP conjugated (Abcam, ab6721, UK); Clarity western ECL substrate (Bio Rad, cat # 170-5061, USA); G-Box Chemi XT Imaging System (Syngene, UK);

### Buffers

1x PBS
   1x TBS Dilute 10x the stock solution in distilled water
   1x TBS-Tween 0.05% (TBST) Dilute 10x the TBS stock solution in distilled water and add Tween.
   Lysis buffer Final concentration of both protein inhibitors is 1:100, diluted in 1x PBS.

### Apparatus for ex-vivo brain slice assay

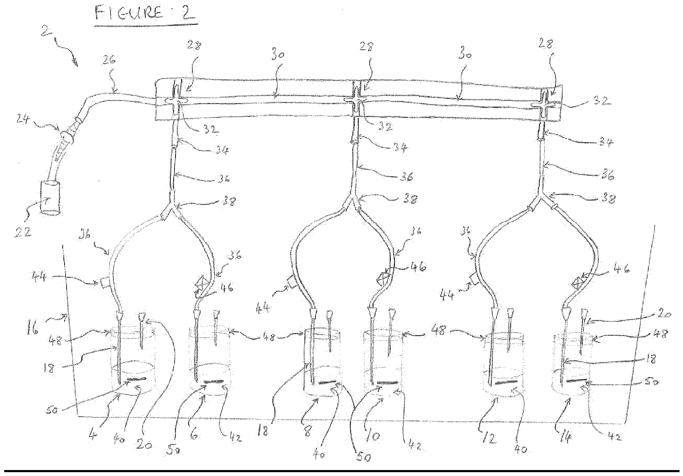

Referring to Figure 2, there is shown an apparatus 2 for carrying out an ex vivo brain slice 50 assay. The apparatus 2 includes a plastic support box 16 in which a series of six 5ml Schott tubular glass Injection vials 4, 6, 8, 10, 12, 14 (Adelphi Healthcare Packaging, VC005-20C, UK) are contained. The vials are divided into two groups of three, i.e. three control group vials 4, 8, 12, which are labelled 44, and three treated group vials 6, 10, 14, which are labelled 46. Each of the control vials 4, 8, 12 contain just artificial cerebrospinal fluid (aCSF) in which a different section of brain 50 is incubated (i.e. rostral, intermediate or caudal), and each of the treated vials 6, 10, 14 contain aCSF in which a different section of brain 50 is incubated (i.e. rostral, intermediate or caudal) with a test compound, such as T30 (SEQ ID No: 3), NBP14 (SEQ ID No: 4) or the peptidomimetic Tri01.

| **Vial** | **Contents** |
|---|---|
| 4 | Brain hemisection including rostral region of basal forebrain in aCSF |
| 6 | Brain hemisection including rostral region of basal forebrain in aCSF + test compound (e.g. T30, NBP14, Tri01) |
| 8 | Brain hemisection including intermediate region of basal forebrain in aCSF |
| 10 | Brain hemisection including intermediate region of basal forebrain in aCSF + test compound (e.g. T30, NBP14, Tri01) |
| 12 | Brain hemisection including caudal region of basal forebrain in aCSF |
| 14 | Brain hemisection including caudal region of basal forebrain in aCSF + test compound (e.g. T30, NBP14, Tri01) |

The vials are each sealed with a 20 mm injection stopper 48 (Adelphi Healthcare Packaging, INJW20RTS, 1071, UK), through which two hypodermic needles 18, 20 extend. The first needle 18 is a 21G bore oxygenating needle by which oxygen is supplied, and the second needle 20 is a 25G bore venting needle through which excess oxygen is vented to atmosphere (BD Microlance, UK).

An oxygen gas store 22 is connected to a series of three air flow control valves 28 and associated taps 32 (Altec Products Ltd, Uk) via 5mm bore PVC tubing 26 (Altec Products Ltd, Uk cat# 01-94-1584), and a straight barbed connector 24 (Cole-Parmer, Uk). Oxygen is fed from the first valve 28 under the control of tap 32 through 3mm bore tubing 34 (cat# 01-94-1580) and then through 1.5 mm bore tubing 36 (cat# 116-0536-19) to a barbed Y connector 38. From the connected 38, a first length of 1.5mm bore tubing 36 feeds to the 21G oxygenating needle 18 in control vial 4, and a second length of 1.5mm bore tubing 36 feeds to the 21G oxygenating needle 18 in treated group vial 6. Passageway 30 connects the three valves 28, 32 together, such that oxygen is fed eventually to vials 8, 10, 12, 14 via the 21G oxygenating needles 18.

The apparatus 2 is assembled as follows with reference to Figures 1 and 2. Referring to Figure 1K, first the air flow control valves 28 are fixed to the container 16 using bundling ties. Connect to one end of the air flow control valves 28, the 5 mm bore tubing 26, which is linked to the oxygen source 22 through the straight barbed connector 24 (Figure 1L). Attach to the lower outlet of the valves 28 the 3 mm bore tubing 34 (Figure 1K). After, insert the three barbed Y connectors 38 in the other side of the 3 mm bore tubing 36. Attach the small tubes 36 to each Y shaped connector 38, in order to have two tubing per each valve (Figure 1K). The free edges of these tubes 36 are glued to 21G needles 18, which are individually inserted in the vials (Figure 1J), where also the 25G needles 20 are placed. The 25G needles 20 enable the outflow of oxygen excess from the vials. Once all the vials (Figure 1I-K) are closed, they are positioned in the 16 and the experiment can start as discussed below (Figure 1L).

In another embodiment, the apparatus 2 is used to study neuroregeneration instead of neurodegeneration. In such a case, instead of using a toxic compound in the containers, a trophic factor such as nerve growth factor (NGF), brain derived neurotrophic factor (BDNF), fibroblast growth factors (FGFs) or T14 peptide can be added to stimulate neuroregeneration.

### Stepwise Procedures

### Brain dissection, slicing and experimental procedure

Referring to Figure 1, the rats were anaesthetized with isoflurane, 15 ml 100% w/w, poured on the cotton layer inside the induction chamber. After testing the proper level of anaesthesia, checking the absence of the pedal reflex, the animals were decapitated, the brains were quickly and carefully extracted (Figure 1A-C) and cooled down in ice-cold "slicing" aCSF for approximately 1-2 minutes. Briefly, after decapitation the skin covering the skull was cut with a surgical blade and the two sides kept under the skull with the index and thumb finger (Figure 1A-B). Then, the skull was cut along the midline with dissection scissors towards the olfactory bulbs and removed with forceps pulling laterally the two halves (Figure 1B-C). After, each brain 50 was gently taken out using a spatula, kept hydrated with ice-cold "slicing" aCSF and placed on filter paper where the cerebellum was cut out with a surgical blade. Subsequently, the brain 50 was vertically glued in the center of the vibratome disc (Figure 1D), placed into the vibratome chamber which was filled with ice cold "slicing" aCSF and surrounded by ice (Figure 1E).

After regulating the cutting distance (Figure 1F), the brain was sectioned (frequency at 6-7 and speed at 6) in three consecutive coronal sections (300 µm thick) containing BF structures (i.e. the rostral, the intermediate and the caudal region of the basal forebrain) (approximately Bregma 0.70 - 0.20 mm) [16] (Figure 1G and 3A). In particular, following the rostro-caudal axis, the sections include the medial septum (MS), diagonal band of Broca (DB) and, to a lesser extent, the substantia innominata (SI), containing the nucleus basalis of Meynert (NBM). Each slice was subsequently cut along the midline (Figure 1G), in order to obtain two hemisections, used respectively as control and treated preparations at the same anatomical level (Figure 3A). All hemisections were then individually placed in 5 ml glass vials (Figure 1H), containing a disc of filter paper, to prevent direct contact between the brain tissue and the glass, and 3 ml of "recording" aCSF alone for the control group (Figure 1I, green frame) or enriched with 2 µM of T30 (or NBP14 or Tri01) for the treated group (Figure 1I, red frame).

The hemisections were positioned into their corresponding vials using two different brushes, one for the control group and the other for the treated counterpart, in order to avoid any contamination. Afterwards, the vials were sealed (Figure 1J-L) and oxygen (95% O₂- 5% CO₂) was continuously provided during the experiment (Figure 1L). The procedure described in relation to Figure 1 was performed within 10-15 minutes to minimize any degradation process of the tissue. The oxygen flow was frequently checked, to prevent hypoxia, and kept at the minimum to avoid the fluctuation of the hemisections, which could be damaged by the oxygenating needle. After 5 hours incubation, the hemislices were separately transferred, with the related brushes (one per condition), into 1.5 ml tubes containing lysis buffer. The brain tissue was then homogenized, keeping the tubes on ice, with pestles. To prevent any contamination between samples, each hemisection was processed with a different pestle. The brain lysate was then centrifuged at 1000 g for 5 minutes at 4°C. The supernatant was transferred to a new tube and stored at -80°C until use.

### Western blot analysis

Protein concentration was determined with Pierce Assay. Aliquots containing 10 µg/µl were prepared mixing the brain lysate with loading buffer and the denaturing agent beta-mercaptoethanol. Samples were heated at 95°C for 5 minutes and stored at -80°C until use. Western blot procedure was performed as previously described [11]. Briefly, proteins were separated through 4-20% precast gels and blotted on PVDF membranes. After staining the membranes with Blot-FastStain to determine the quality of the transfer and the protein level, the blots were destained with warm distilled water until all the bands were removed. Then the blots were blocked in 5% blotting grade blocker in TBS for 1 hour at room temperature (RT). Once finished the blocking step, the blots were rinsed 3 x 5 minutes with TBST and incubated overnight at 4°C with primary antibodies diluted with 1% blocking solution prepared with TBST. The next day, the membranes were thoroughly washed in TBST 5 x 5 min, then incubated for one hour at RT with the secondary antibodies, diluted in TBST. After the incubation, the blots were rinsed 6 x 5 min with TBST and 10 min with TBS and probed for protein determination using chemiluminescent substrates. The primary antibodies, all diluted at 1:1000, were: mouse anti-phosphorylated Tau, mouse anti-Amyloid beta and rabbit anti-α7-nAchR. The secondary antibodies, all HRP conjugated, were goat anti mouse (1:2000) and goat anti rabbit (1:5000).

### Statistical analysis

Protein values were normalized to total protein expression [17] and analyzed with ImageJ software (NIH, USA). Differences in protein levels between control and treated group were determined using paired Student's t-test and subsequently plotted with Graphpad software (Graphpad Prism 6, San Diego, USA). All data were considered significant with a *p-value* < 0.05.

### Troubleshooting

Table 1 indicates possible issues which can occur during the procedure, their related cause and suggestions to avoid them.

**Table 1 - Problems and solutions for the apparatus and assay/model**

| **Problem** | **Possible cause** | **Solution** |
|---|---|---|
| Poor quality of the tissue | Mechanical damage during brain removal or slicing. | Operate gently and precisely while handling the tissue. |
| | Slow dissecting and slicing. | Train to dissect out and process faster the brain. |
| | The tissue is not kept cold. | Ensure that the brain is maintained on ice cold aCSFs during removal and sectioning. |
| | The hemisections are floating inside the vials. | Reduce the oxygen flow and/or move away the "oxygenating" needle from the top of the hemislice to avoid a direct contact which can impair the integrity of the tissue. |
| | Old solutions. | |
| | Anoxia during the experiment. | Prepare frequently fresh solutions. |
| | | Check whether the slices are constantly oxygenated. |
| Inconstant oxygenation | The oxygen canister is empty. | Check before starting the experiment if there is enough oxygen. |
| | The valves are closed. | Control the canister and in particular the apparatus valves and open them before starting. |
| | The tubing are not properly connected or broken. | Check periodically the integrity and functionality of all the components and if requested change them. |
| | The "oxygenating" needle is plugged. | Remove quickly the injection stopper from the vial and flick the needle. Probably a bubble air is blocking the oxygen flow. |
| | The "oxygenating" needle is not immersed in the aCSF. | Ensure that the needle is properly immersed within the aCSF. |

### Timing

- Brain removal: within 1 minute (min);
- Brain preparation for the slicing (attachment to the vibratome disc and fixation inside the slicing chamber): 1-2 min;
- Slicing: within 10 min;
- Transfer of the brain hemislices in the glass vials and starting the experiment: 1-2 min. During this time the slices are not oxygenated until all the lids are closed and the apparatus is connected to the oxygen source, so this step should be as fast as possible;
- Experiment length: 5 hours;
- Tissue transfer into the 1.5 ml tubes and homogenization: 10 min;
- Centrifugation of the brain lysate: 5 min;
- Transfer of the surpernatant in new tubes: 1-2 min;
- The time in each step, excluding the 5 h incubation and centrifugation, can vary depending on the experience of the researcher.

### Results

*T30 treatment affects T14*, *α7-nAchR, Aβ and p-Tau levels in a site-specific manner* After 5 hours, T30 exposure (Figure 3A) induced a heterogeneous expression of T14 Figure 3B), α7-nAChR (Figure 3C), Aβ (Figure 3D) and p-Tau (Figure 3E). Of particular note, was the observation that the levels of these proteins change along a rostro-caudal axis. The endogenous T14 expression was not significantly affected after T30 administration, when comparing the two conditions (Figure 3B).
The α7 receptor, showed a 51% increase in the rostral portion (slice 1, p=0.02) in the treated group (Figure 3C), whereas in the intermediate slice there was no difference in the two groups (slice 2, p=0.39) (Figure 3C). In the caudal section its levels were reduced by 53% compared to the control group (slice 3, p=0.001) (Figure 3C).

Aβ levels were increased in all the T30 treated sections. However, in the anterior portion the difference between the two conditions was not significant, 46% (slice 1, p=0.33), whereas in the remaining slices, an increase by 87% (slice 2, p=0.01) and 98% (slice 3, p=0.02) was observed in the treated sides above the controls (Figure 3D).

p-Tau displayed higher levels, 90%, in the rostral hemisection upon T30 application, (slice 1, p=0.05) (Figure 3E), while the other slices did not show any change in the two groups (slice 2, p=0.37; slice 3, p=0.74) (Figure 3E).

### Discussion

The advantages of the apparatus and ex vivo assay described herein are that they provide the opportunity to monitor, in a time and site-dependent manner, key neurochemical events potentially underlying neuronal dysfunction in a preparation that combines the advantages of an *in-vivo* physiological milieu, comprising preserved brain cytoarchitecture and functional local synaptic network, with those of *in-vitro,* such as precision, accessibility and control of the extracellular environment.

This *ex-vivo* model is an adaptation of the common brain slice method for real-time recordings, widely used for electrophysiology or optical imaging [15,18,19]. The goal was to evaluate the T30 effects, over a far longer period of time (hours) and in a more physiological context than tissue culture [11,13], on contributing to slower neuronal processes characterizing the pathophysiology of neurodegenerative disorders.

The data presented here indicate that, following T30 administration, a distinct anatomical expression of T14, alpha7 nicotinic acetylcholine receptor, amyloid beta and p-Tau occurs. The AChE-peptide showed no differences in all the three portions after comparison with their control counterparts. (Figure 3B). The nicotinic receptor showed an increase in the rostral treated side and a decrease in the caudal one compared to the untreated group (Figure 3C, slice 1 and 3), whereas the intermediate region does not show any change between the two conditions (Figure 3C, slice 2). Aβ was constantly overexpressed in all the T30 exposed hemisections (Figure 3D), showing a significant increase in the medial and caudal region above the control levels (Figure 3D, slice 2 and 3).
On the other hand, phosphorylated Tau levels were higher only in the rostral treated hemisection compared to the control counterpart (Figure 3E, slice 1), while no variation was observed in the other two slices comparing the two conditions (Figure 3E, slice 2 and 3).
These results suggest that the AChE-peptide could exert a pivotal role in varying the protein expression along the rostro-caudal axis (Table 2), since, in contrast, in all the controls the protein levels are similar (Figure 3B-E).

**Table 2 - Anatomical specificity in protein expression induced by T30 administration**

| The levels of each protein were indicated comparing the amount observed in the treated sides and their control counterparts following the rostro-caudal path of the region of interest. +, -, = indicate a significant increase, decrease or no change in protein expression in the treated group (T30, NBP14 etc..) compared to the normalized control values, indicated with 1 | | | | | | |
|---|---|---|---|---|---|---|
| **AD hallmarks** | **Slice 1 (rostral)** | | **Slice 2 (intermediate)** | | **Slice 3 (caudal)** | |
| | ctrl 1 | T30 1 | ctrl 2 | T30 2 | ctrl 3 | T30 3 |
| α7-nAChR | 1 | + | 1 | = | 1 | - |
| p-Tau | 1 | + | 1 | = | 1 | = |
| Aβ | 1 | = | 1 | + | 1 | + |

These observations are consistent with former studies describing a T30 mediated upregulation of the α7-nAChR [13]. As a consequence, the influx of calcium into each sub-division, through a heterogeneous density of the α7-nAChR, may in turn favor p-Tau or Aβ pathways. In any event, the data are in line with earlier evidence indicating a functional interaction involving this receptor with p-Tau and Aβ [20-22]. Furthermore, several findings indicate that the basal forebrain nuclei are not homogenous in their features: they display a diverse neuronal morphology [23], as well as a differential anteroposterior distribution of GSK-3β [24], which is indirectly activated by T30 [13] and regulates Tau phosphorylation or Aβ generation. In addition, these molecular mechanisms might determine cell death, which in AD occurs within the BF in a site specific manner [8,25]. Taken all together, these findings would corroborate the heterogeneous expression of the proteins described in this study.

The inventors have therefore demonstrated that T30 administration on *ex-vivo* brain slices containing BF alters, in a site-specific way, the expression of T14, andα7-nAChR, which may in turn contribute to the modulation of p-Tau and Aβ.

Beyond the validation of the specific hypothesis explored here, this technique could open up a valuable approach to investigate a wide range of neuropathological processes, since it preserves the cytoarchitecture, the neuronal matrix and local circuitry of a particular brain region over a sustained time window, thereby giving a direct and sensitive read-out of a specific level of the brain under physiological and indeed pathological conditions.

### Advantages of the protocol

The apparatus and model provide several advantages:
1) They maintain the anatomical site-selectivity and the physiological milieu of the living brain;
2) Slices can be easily obtained for studying a specific area;
3) Direct intervention, precision dosing, and an accurate control by comparing drug-treated and untreated contralateral side from the same slice;
4) The potential reduction in the number of living animals ultimately used in behavioural studies.

### Example 2 - Use of NBP14 in ex vivo assay/model

Following the results in Example 1 using toxic T30, the inventors then tested the cyclic T14 peptide, NBP14 (SEQ ID No:4), on *ex-vivo* brain slices using precisely the same procedure adopted with T30. Hence, each of the control vials 4, 8, 12 contained just artificial cerebrospinal fluid (aCSF) in which a different section of brain is incubated (i.e. rostral, intermediate or caudal), and each of the treated vials 6, 10, 14 contained aCSF in which a different section of brain is incubated (i.e. rostral, intermediate or caudal) with NBP14.

The results are shown in Figure 4. In general, in all of the protein investigated, the administration of NBP14 does not affect the protein levels which are comparable to the control one. Interestingly, this pattern is opposite when compared to T30, since it triggers a different protein expression in the treated group compared to the untreated counterpart. In the table below, the effects of T30 are compared to those of NBP14. Looking at each protein, it shows how the two compounds act in a distinct manner, since NBP14 reverses or prevents the negative effects seen with T30 application.

**Table 3 - Summary of T30 and NBP14 related effects along the antero-posterio plane of brain slices including the basal forebrain**

| Markers/Compounds | T30 | NBP14 |
|---|---|---|
| T14 | Increasing trend | Decreasing trend |
| a7-nAChR | Decreasing trend | No change |
| Aβ | Increasing trend | Decreasing trend |
| p-Tau | Decreasing trend | No change |

### Example 3 - Preparation and use of Tri 01 in ex vivo assay/model

### Materials and Methods

The peptidomimetic, Tri 01, was synthesised by Genosphere Biotechnologies and analysed for purity using RP-HPLC (>99% pure), and mass by mass spectroscopy. Referring to Figure 5, there is shown the structure of Tri01, and its affinity for the T14 site on the alpha 7 nicotinic receptor.
Tri_01:

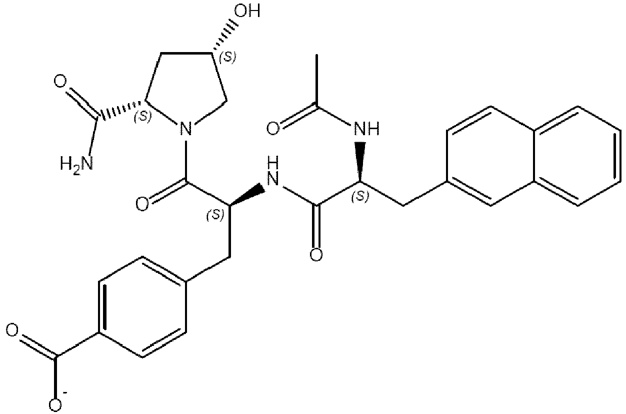

### 4-((S)-2-((S)-2-acetamido-3-(naphthalen-2-yl)propanamido)-3-((2S,4S)-2-carbamoyl-4-hydroxypyrrolidin-1-yl)-3-oxopropyl)benzoate

### Score: -10.3

### Brief stepwise description of synthesis of TRI 01 - Sequence: [acetyl]-[2Nal][4cooh-F][hydroxyproline]-[amide]

### Brief stepwise description of synthesis:

1) Rink Amide MBHA. Resin Soak in DCM for 30mins, pumped dry, washed by DMF for 3 times, pumped dry.
2) Add Fmoc-Hyp(tBu)-OH, DIEA, TBTU, DMF, N2, reaction for 2 h, pumped dry, washed by DMF for 6 times, pumped dry.
3) Add piperidine/DMF to remove Fmoc-, reaction for 20mins, pumped dry, washed by DMF for 3 times, pumped dry.
4) Add Fmoc-Phe(4-COOHtBu)-OH, DIEA, TBTU, DMF, N2, reaction for 2 h, pumped dry, washed by DMF for 6 times, pumped dry.
5) Repeat step 3.
6) Add Fmoc-2Nal-OH, DIEA, TBTU, DMF, N2, reaction for 2h, pumped dry, washed by DMF for 6 times, pumped dry.
7) Repeat step 3.
8) Add Acetic Anhydride /pyridine, N2, reaction for 30 mins, pumped dry, washed by DMF for 3 times, pumped dry, washed by DCM for 3 times, pumped dry, washed by MeOH for 3 times, pumped dry.
9) Peptide cleaved from resin, pumped dry, precipitation reaction by diethyl ether, obtain the crude peptide, centrifugal drying.
10) Purification

### Results

### Ex vivo brain slices

Referring to Figure 6, there is shown the results on *ex-vivo* brain slices with or without Tri01 treatment. As can be seen, the markers evaluated through WB analysis, i.e. T14, Tau and Aβ, did not show any change in their levels comparing the control group with the treated one, along the antero-posterior plane. However, Tri01 is believed to exert a toxic effect since it promotes an increase of APP, which is the precursor of amyloid beta.

### Cell viability test

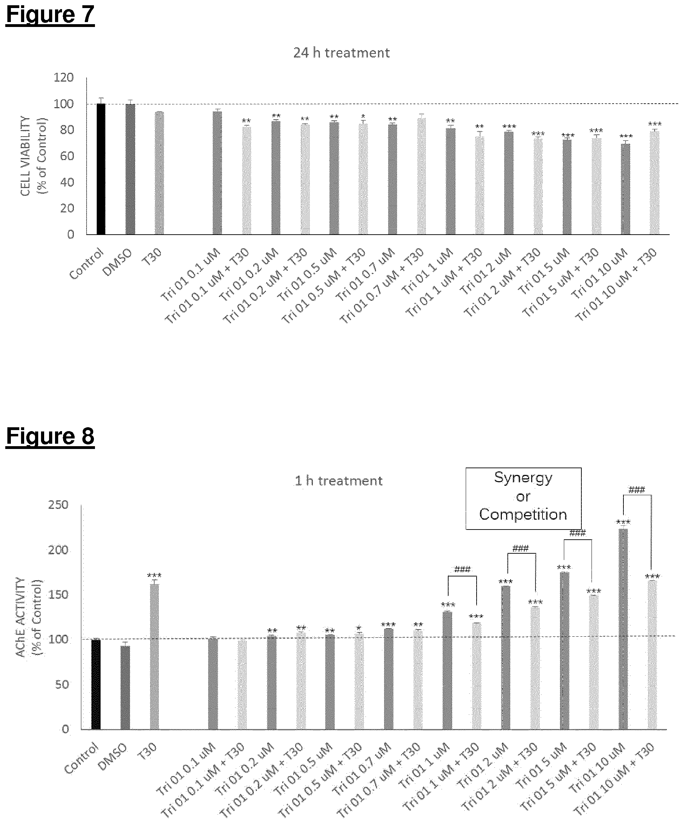

Tri01 alone was toxic in a concentration-dependent manner (0.1µM to 10µM). Moreover, it potentiates the toxicity induced by T30 1µM, also in a concentration-dependent manner, as shown in Figure 7.

### AChE activity test

As shown in Figure 8, Tri01 alone induced an increase in AChE activity in a concentration-dependent manner (0.1µM to 10µM). Contrarily to the cell viability test, the effect of Tri01 was reduced in the presence of T30 1µM, suggesting a synergy or a competition on the alpha7 nicotinic receptor.

### Calcium influx test

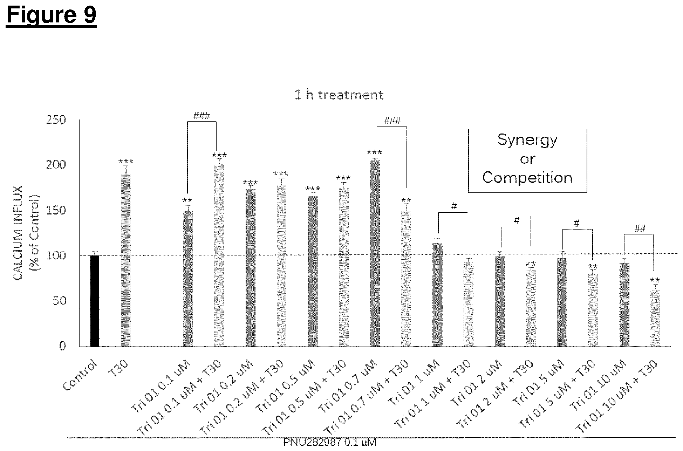

Referring to Figure 9, Tri01 alone acts as an allosteric modulator (0.1 µM to 0.7 µM) potentiating calcium influx by the specific alpha7 agonist PNU282987, but at higher doses (1 µM to 10 µM) it becomes inactive. On the other hand, in the presence of T30, at the high doses the 2 molecules synergise by blocking the receptor. This effect of antagonism is due to the overstimulation of the receptor by the two molecules that induces its desensitation and it shuts down its calcium channel.

### Conclusions

In PC12 cells:
- Tri01 decreases cell viability;
- Tri01 increases AChE activity;
- Tri01 induces calcium influx;
- Tri01 has a dose-dependent synergistic effect with T30 visible on AChE and calcium tests;
- Tri01 could be competing with the T30/T14 site on the receptor;
- Tri01 is toxic and not a good candidate to protect from T30 toxicity;
- Tri01 is a good candidate as a toxic molecule that acts like T30 in the animal model of Alzheimer's disease, Parkinson's disease or Motor Neurone Disease.

### Summary

The inventors have developed a novel ex vivo rodent brain slice model or assay for the analysis of neurodegeneration in a manner which very closely reproduces the full pathological profile (e.g. of Alzheimer's disease). The apparatus and model can be used to study neurodegeneration in a more physiological scenario (i.e. *ex-vivo* brain slices) evaluating the expression of several markers, such as α7-nAChR, p-Tau and Aβ, whose expression is altered during neurodegenerative events. The inventors have also designed, built and tested a novel apparatus for inducing and monitoring neurodegeneration. The apparatus and assay can therefore be used to test novel agents which may exhibit neuroprotective or neurotoxic activity. Thus, the apparatus and models can be used to examine many molecular processes, test pharmacological compounds which may regulate these processes and provide a reliable tool for drug screening, reducing whole animal experiments.

In addition, the inventors have designed, synthesised and tested the novel peptidomimetic, Tri01, which has been shown to be toxic, because it (i) decreases cell viability, (ii) increases AChE activity, and (iii) induces calcium influx. As such, like T30, Tri01 could be used in the assay to cause neurodegeneration, and against which novel compounds could be tested to see if they can cease or even reverse the neurodegeneration, similar to NBP14.

### References

[1] De Strooper B, Karran E. The Cellular Phase of Alzheimer's Disease. Cell 2016;164:603-15. doi:10.1016/j.cell.2015.12.056.
[2] Small GW, Greenfield S. Current and Future Treatments for Alzheimer Disease. Am J Geriatr Psychiatry 2015;23:1101-5. doi:10.1016/j.jagp.2015.08.006.
[3] Arendt T, Brückner MK, Lange M, Bigl V. Changes in acetylcholinesterase and butyrylcholinesterase in Alzheimer's disease resemble embryonic development-A study of molecular forms. Neurochem Int 1992;21:381-96. doi: 10.1016/0197-0186(92)90189-X.
[4] Arendt T, Bruckner MK, Morawski M, Jager C, Gertz HJ. Early neurone loss in Alzheimer's disease: cortical or subcortical? Acta Neuropathol Commun 2015;3:10. doi: 10.1186/s40478-015-0187-1.
[5] Auld DS, Kornecook TJ, Bastianetto S, Quirion R. Alzheimer's disease and the basal forebrain cholinergic system: relations to β-amyloid peptides, cognition, and treatment strategies. Prog Neurobiol 2002;68:209-45. doi:10.1016/S0301-0082(02)00079-5.
[6] Mesulam M, Shaw P, Mash D, Weintraub S. Cholinergic nucleus basalis tauopathy emerges early in the aging-MCI-AD continuum. Ann Neurol 2004;55:815-28. doi:10.1002/ana.20100*.*
[7] Schliebs R, Arendt T. The cholinergic system in aging and neuronal degeneration. Behav Brain Res 2011;221:555-63. doi:10.1016/j.bbr.2010.11.058.
[8] Schmitz TW, Nathan Spreng R, Weiner MW, Aisen P, Petersen R, Jack CR, et al. Basal forebrain degeneration precedes and predicts the cortical spread of Alzheimer's pathology. Nat Commun 2016;7:13249. doi:10.1038/ncomms13249.
[9] Mesulam MM, Mufson EJ, Wainer BH, Levey AI. Central cholinergic pathways in the rat: An overview based on an alternative nomenclature (Chi-Ch6). Neuroscience 1983;10:1185-201. doi:10.1016/0306-4522(83)90108-2.
[10] Greenfield S. Discovering and targeting the basic mechanism of neurodegeneration: The role of peptides from the C-terminus of acetylcholinesterase: Non-hydrolytic effects of ache: The actions of peptides derived from the C-terminal and their relevance to neurodegeneration. Chem Biol Interact 2013;203:543-6. d01:10.1016/j.cbi.2013.03.015.
[11]Garcia-Ratés S, Morrill P, Tu H, Pottiez G, Badin AS, Tormo-Garcia C, et al. (I) Pharmacological profiling of a novel modulator of the α7 nicotinic receptor: Blockade of a toxic acetylcholinesterase-derived peptide increased in Alzheimer brains. Neuropharmacology 2016;105:487-99. doi: 10.1016/j.neuropharm.2016.02.006.
[12] Greenfield S, Vaux DJ. Commentary Parkinson'S Disease, Alzheimer's Disease and Motor Neurone Disease: Identifying a Common Mechanism. Science (80-)2002;113:485-92.
[13] Bond CE, Zimmermann M, Greenfield SA. Upregulation of α7 nicotinic receptors by acetylcholinesterase C-terminal peptides. PLoS One 2009;4:34-6. doi:10.1371/journal.pone.0004846.
[14] Greenfield SA, Day T, Mann EO, Bermudez I. A novel peptide modulates a 7 nicotinic receptor responses : implications for a possible trophic-toxic mechanism within the brain 2004:325-31. doi:10.1111/j.1471-4159.2004.02494.x.
[15] Badin AS, John E, Susan G. High-resolution spatio-temporal bioactivity of a novel peptide revealed by optical imaging in rat orbitofrontal cortex in vitro: Possible implications for neurodegenerative diseases. Neuropharmacology 2013;73:10-8. doi:10.1016/j.neuropharm.2013.05.019.
[16] Paxinos G. W. The Rat Brain in stereotaxic coordinates. Fourth edi. San Diego, USA: Elsevier; 1998.
[17] Collins MA, An J, Peller D, Bowser R. Total protein is an effective loading control for cerebrospinal fluid western blots. J Neurosci Methods 2015;251:72-82. doi:10.1016/j.jneumeth.2015.05.011.
[18] Llinas RR, Greenfield SA. On-line visualization of dendritic release of acetylcholinesterase from mammalian substantia nigra neurons. ProcNatlAcadSci USA 1987;84:3047-50.
[19] Badin AS, Morrill P, Devonshire IM, Greenfield SA. (II) Physiological profiling of an endogenous peptide in the basal forebrain: Age-related bioactivity and blockade with a novel modulator. Neuropharmacology 2016;105:47-60. doi: 10.1016/j.neuropharm.2016.01.012.
[20] Dineley KT, Bell KA, Bui D, Sweatt JD. β-amyloid peptide activates α7 nicotinic acetylcholine receptors expressed in Xenopus oocytes. J Biol Chem 2002. doi: 10.1074/jbc.M200066200.
[21] Rubio a, Perez M, Avila J. Acetylcholine receptors and tau phosphorylation. Curr Mol Med 2006;6:423-8.
[22] Wang H-Y, Lee DHS, D'Andrea MR, Peterson PA, Shank RP, Reitz AB. β-Amyloid1-42 Binds to α7 Nicotinic Acetylcholine Receptor with High Affinity. J Biol Chem 2000;275:5626-32.
[23] Dinopoulos A, Parnavelas JG, Uylings HB, Van Eden CG. Morphology of neurons in the basal forebrain nuclei of the rat: a Golgi study. J Comp Neurol 1988;272:461-74.
[24] Leroy K, Brion J-P. Developmental expression and localization of glycogen synthase kinase-3β in rat brain. J Chem Neuroanat 1999;16:279-93. doi: 10.1016/S0891-0618(99)00012-5.
[25] Liu AKL, Chang RCC, Pearce RKB, Gentleman SM. Nucleus basalis of Meynert revisited: anatomy, history and differential involvement in Alzheimer's and Parkinson's disease. Acta Neuropathol 2015;129:527-40. doi:10.1007/s00401-015-1392-5.

## Claims

1. An apparatus for carrying out an animal model for a neurodegenerative disease or neuroregeneration, the apparatus comprising:-
- a first container configured to receive a first brain section from an animal brain, and comprising incubation media for retaining viability of the brain section;
- a second container configured to receive a second brain section, which is contralateral with the first brain section, and comprising incubation media for retaining viability of the brain section; and
- oxygen feed means configured to feed oxygen into the media in the first and second containers to thereby incubate the first and second brain sections to retain their viability, wherein the incubation media in at least one of the containers comprises a compound which contributes to, or causes, neurodegeneration or neuroregeneration of the brain section contained therein.

2. An apparatus according to claim 1, wherein the animal model is an *ex-vivo* model.

3. An apparatus according to either claim 1 or claim 2, wherein the apparatus is used to investigate any neurodegenerative disease selected from a group consisting of: including Alzheimer's disease; Parkinson's disease; Huntington's disease; Motor Neurone disease; Spinocerebellar type 1, type 2, and type 3; Amyotrophic Lateral Sclerosis (ALS); schizophrenia; Lewy-body dementia; and Frontotemporal Dementia, preferably wherein the model is used to study Alzheimer's Disease.

4. An apparatus according to any preceding claim, wherein the brain is a non-human mammalian brain, such as a rodent brain, and/or wherein the media comprises cerebrospinal fluid (CSF), most preferably artificial cerebrospinal fluid (aCSF).

5. An apparatus according to any preceding claim, wherein the first and second brain sections comprise the basal forebrain, preferably wherein:
- the first and second brain sections each comprise contralateral hemisections comprising the basal forebrain; and/or
- the first and second brain sections are selected from a group consisting of: the rostral region of the basal forebrain; the intermediate region of the basal forebrain; and the caudal region of the basal forebrain; and/or
- the first and second brain sections comprise contralateral hemisections comprising the rostral region of the basal forebrain, or the first and second brain sections comprise contralateral hemisections comprising the intermediate region of the basal forebrain, or the first and second brain sections comprise contralateral hemisections comprising the caudal region of the basal forebrain.

6. An apparatus according to any preceding claim, wherein the apparatus comprises four containers, wherein two pairs of containers are configured to each receive contralateral sections of two different regions of the basal forebrain, preferably wherein the apparatus comprises six containers, wherein three pairs of containers are configured to each receive contralateral sections of three different regions of the basal forebrain.

7. An apparatus according to any preceding claim, wherein the apparatus comprises: -
- a first container configured to receive a first brain section comprising the rostral region of the basal forebrain, and a second container configured to receive a second contralateral brain section also comprising the rostral region of the basal forebrain; and/or
- a third container configured to receive a third brain section comprising the intermediate region of the basal forebrain, and a fourth container configured to receive a fourth contralateral brain section also comprising the intermediate region of the basal forebrain; and/or
- a fifth container configured to receive a fifth brain section comprising the caudal region of the basal forebrain, and a sixth container configured to receive a sixth contralateral brain section also comprising the caudal region of the basal forebrain.

8. An apparatus according to any preceding claim, wherein the oxygen feed means comprises a source of oxygen gas, and one or more conduits and associated valves, which are configured to feed oxygen into the incubation media that is present in each container, preferably wherein one end of the conduits comprises a needle which extends into the incubation media in the container.

9. An apparatus according to any preceding claim, wherein each container comprises a vent through which excess oxygen may be vented to atmosphere.

10. An apparatus according to any preceding claim, wherein the compound, which contributes to, or causes, neurodegeneration of the brain section, is an allosteric modulator of the α7 nicotinic receptor.

11. An apparatus according to any preceding claim, wherein the compound comprises a polypeptide, derivative or analogue thereof, comprising or consisting of an amino acid sequence derived from the C-terminus of acetylcholinesterase (AChE), or a truncation thereof, preferably wherein the acetylcholinesterase comprises an amino acid sequence substantially as set out in SEQ ID No:1, preferably excluding the 31 amino acids at the N-terminal, more preferably wherein the compound, which contributes to, or causes, neurodegeneration of the brain section comprises or consists of SEQ ID No: 3, 4 or 5.

12. An apparatus according to any one of claims 1 to 10, wherein the compound, which contributes to, or causes, neurodegeneration of the brain section, is a compound having Formula (I): 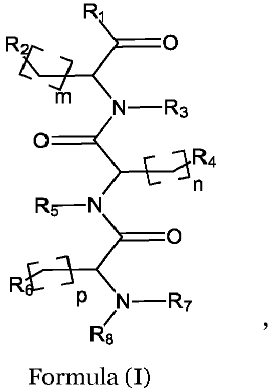 wherein:
R₁ is -NR₉R₁₀ or -OH;
R₂ is -NH₂, -OH, -N(H)C(NH)NH₂, -C(O)OH or -C(O)NH₂;
R₃ is -H or a C₁₋₅ straight or branched alkyl or alkenyl; or
R₂ and R₃ together with the nitrogen and carbon(s) to which they are bonded form a ring substituted by -OH or -NH2;
R₄ is aryl or heteroaryl, optionally substituted with one or more X₁ groups;
R₅ is -H or a C₁₋₅ straight or branched alkyl or alkenyl;
R₆ is aryl or heteroaryl, optionally substituted with one or more X₁ groups;
R₇ is -H or a C₁₋₅ straight or branched alkyl or alkenyl;
R₈ is -H, a C₁₋₅ straight or branched alkyl or alkenyl or -C(O)R₁₂;
the or each X₁ group is independently selected from -NR₉R₁₀, - OH or -C(O)R₁₂;
the or each R₉ and R₁₀ are independently -H or a C₁₋₅ straight or branched alkyl or alkenyl;
R₁₁ is -NH2, -OH or an aryl group;
R₁₂ is a C₁₋₅ straight or branched alkyl or alkenyl; and
m, n and p are each independently between 0 and 10;
or a salt, solvate, tautomeric form, or polymorphic form thereof.

13. A method of providing an animal model for a neurodegenerative disease or neuroregeneration, the method comprising:-
- removing a region of an animal brain, and dividing the brain region into corresponding first and second contralateral brain sections;
- incubating the first and second brain sections to retain their viability; and
- contacting at least one of the brain sections with a compound which contributes to, or causes, neurodegeneration or neuroregeneration of the brain section.

14. Use of the apparatus according to any one of claims 1-12 to: (i) examine neurodegenerative processes; (ii) test pharmacological compounds which may modulate neurodegenerative processes; or (iii) screen neurodegenerative drugs.

15. A method of identifying an agent that modulates neurodegeneration or a candidate agent, for use in the treatment, prevention or amelioration of neurodegenerative disorder, the method comprising:
- removing a region of an animal brain, and dividing the brain region into corresponding first and second contralateral brain sections;
- contacting each brain section with a compound which contributes to, or causes, neurodegeneration of the brain sections;
- contacting one of the brain sections with a test agent prior to, simultaneously or after the compound which causes, or contributes to, neurodegeneration of the brain section; and
- determining whether the test agent modulates neurodegeneration or inhibits or prevents neurodegeneration in the brain section by comparing its effects against the brain section untreated with the test agent, wherein inhibition or prevention of neurodegeneration compared to the control indicates that the test agent is a candidate for the treatment, prevention or amelioration of neurodegenerative disorder.
